# EUROPEAN PATENT APPLICATION

(11) **EP 4 650 489 A1**
(43) Date of publication of application: **19.11.2025**
(21) Application number: 24741544.1
(22) Date of filing: 10.01.2024
(51) Int. Cl.: C25B 9/00, C07B 61/00, C07C 68/00, C07C 69/96, C25B 1/23, C25B 3/07, C25B 3/23, C25B 9/13, C25B 9/60, C25B 11/054, C25B 15/02

(54) **ELECTROCHEMICAL REACTOR AND METHOD FOR PRODUCING OXYGEN-CONTAINING ORGANIC MATERIAL**

(30) Priority: 10.01.2023 JP 2023001986; 26.09.2023 JP 2023163732
(71) Applicant: Sekisui Chemical Co., Ltd., Osaka-shi, Osaka 530-8565 (JP)
(72) Inventor: NISHI, Hiroaki, Tsukuba-shi, Ibaraki 300-4292 (JP); FUJINUMA, Naohiro, Tsukuba-shi, Ibaraki 300-4292 (JP); MATSUMOTO, Daichi, Tsukuba-shi, Ibaraki 300-4292 (JP); IKAWA, Hiroyuki, Tsukuba-shi, Ibaraki 300-4292 (JP); TAKEUCHI, Toshimasa, Tsukuba-shi, Ibaraki 300-4292 (JP)
(74) Representative: Ter Meer Steinmeister & Partner
(86) International application number: PCT/JP2024/000326
(87) International publication number: WO 2024/150772

(57) **Abstract**

An electrochemical reaction apparatus 10 comprises: a cathode 11 that comprises a first catalyst 16 that reduces carbon dioxide to carbon monoxide; an anode 12; an electrolytic solution 13 that contains a reactant and an electrolyte; and a second catalyst 17 that synthesizes an oxygen-containing organic material from the carbon monoxide and the reactant, in which the electrolytic solution 13 has a convection flow, and the direction of the convection flow F is formed so that the electrolytic solution comes into contact with the first catalyst 16 and the second catalyst 17 in this order.

## Description

### Technical Field

The present invention relates to an electrochemical reaction apparatus that produces an oxygen-containing organic material, such as a carbonyl compound, using carbon dioxide as a raw material, and a method for producing an oxygen-containing organic material.

### Background Art

Electrochemical synthesis methods have gained prominence in recent years because they do not require the use of highly toxic substrates or highly explosive oxygen mixed gases, and they can also directly utilize electricity from renewable energy. Furthermore, in recent years, the production of organic materials by electrochemical reactions using carbon dioxide or carbon monoxide, which is obtained by reducing carbon dioxide, as raw materials has been studied for the purpose of suppressing global warming and replacing fossil fuels.

Carbonyl compounds such as organic carbonates have been utilized in a variety of fields, including paints, adhesives, electrolytic solutions, and raw materials for resins, and with regard to carbonyl compounds as well, electrochemical synthesis using carbon dioxide as a raw material and a variety of catalysts has been attempted in recent years.

For example, PTL 1 discloses an electrochemical reaction apparatus comprising: a first electrochemical compartment that is provided with a first electrode; a second electrochemical compartment that is provided with a second electrode and contains an electrolytic substrate; an ion conducting membrane that divides into the first electrochemical compartment and the second electrochemical compartment; and a connecting path that links the first electrochemical compartment and the second electrochemical compartment. In the electrochemical reaction apparatus of PTL 1, carbon dioxide is reduced to carbon monoxide at the first electrode (cathode), and the produced carbon monoxide flows out via a connecting pipe into the second electrochemical compartment, where a valuable product such as an organic carbonate is produced at the second electrode (anode) from the carbon monoxide and an electrolytic solution.

### Citation List

### Patent Literature

PTL 1: WO 2019/182164

### Summary of Invention

### Technical Problem

However, methods for producing carbonyl compounds such as organic carbonates using conventional electrochemical reaction apparatuses are required to be further improved for practical use because of the complicated structure of the electrochemical reaction apparatuses.

Also, in electrochemical reaction apparatuses that produce the target valuable products at the anode using carbon monoxide that has been converted at the cathode, if the current density is increased in order to increase the amount of the target products to be produced, halide ions in the electrolyte react more readily, resulting in the generation of halogen as a byproduct. When halide ions react to become halogen, the electrolyte concentration is decreased, which increases electrical resistance and power consumption, thus increasing the production cost. In addition, the generation of byproduct decreases the amount of the target products to be produced, thereby decreasing the production efficiency. Therefore, there is a need for an electrochemical reaction apparatus that can increase the amount of production while keeping power consumption low when obtaining a valuable product, such as a carbonate compound, from carbon dioxide.

Therefore, an object of the present invention is to provide an electrochemical reaction apparatus that can electrochemically synthesize an oxygen-containing organic material, such as a carbonyl compound, from carbon dioxide by the apparatus that has a relatively simple structure, and a method for producing an oxygen-containing organic material using the electrochemical reaction apparatus.

Also, a more limited object of the present invention is to provide an electrochemical reaction apparatus that can increase the amount of production while keeping power consumption low when obtaining an oxygen-containing organic material, such as a carbonyl compound, from carbon dioxide, and a method for producing an oxygen-containing organic material using the electrochemical reaction apparatus.

### Solution to Problem

The present invention provides the following [1] to [29].
[1] An electrochemical reaction apparatus comprising:
   a cathode comprising a first catalyst that reduces carbon dioxide to carbon monoxide;
   an anode;
   an electrolytic solution comprising a reactant and an electrolyte; and
   a second catalyst that synthesizes an oxygen-containing organic material from the carbon monoxide and the reactant,
   the electrolytic solution having a convection flow, and a direction of the convection flow formed so that the electrolytic solution comes into contact with the first catalyst and the second catalyst in this order.
[2] The electrochemical reaction apparatus according to the above [1], wherein the first catalyst and the second catalyst are arranged on an identical plane, and the electrolytic solution has a convection flow that flows in parallel to the plane.
[3] The electrochemical reaction apparatus according to the above [1], wherein the first catalyst and the second catalyst are arranged so that they face each other in the electrochemical reaction apparatus.
[4] The electrochemical reaction apparatus according to the above [3], comprising a rectifying layer between the first catalyst and the second catalyst.
[5] The electrochemical reaction apparatus according to the above [3], comprising an isolating layer between the first catalyst and the second catalyst that isolates the carbon monoxide generated by the first catalyst from an oxidized product generated by the anode.
[6] The electrochemical reaction apparatus according to the above [1], wherein the first catalyst and the second catalyst are arranged so that they are orthogonal or inclined.
[7] The electrochemical reaction apparatus according to any one of the above [1] to [6], wherein the anode comprises the second catalyst.
[8] The electrochemical reaction apparatus according to any one of the above [1] to [7], wherein the electrochemical reaction apparatus comprises a first reaction section that comprises the cathode and the anode and a second reaction section that comprises the second catalyst, and
   the electrolytic solution has a direction of the convection flow that flows in an order of the first reaction section and the second reaction section.
[9] The electrochemical reaction apparatus according to the above [8], comprising an isolating layer in the first reaction section that isolates the carbon monoxide generated by the first catalyst from an oxidized product generated by the anode.
[10] The electrochemical reaction apparatus according to the above [8] or [9],
   wherein an inside of the first reaction section is isolated into a cathode side region and an anode side region by the isolating layer, and
   the electrolytic solution has a direction of the convection flow that flows in an order of the cathode side region, the anode side region, and the second reaction section.
[11] The electrochemical reaction apparatus according to any one of the above [8] to [10],
   wherein an inside of the first reaction section is isolated into a cathode side region and an anode side region by the isolating layer, and
   the electrolytic solution has a direction of the convection flow that flows in an order from each of the cathode side region and the anode side region to the second reaction section.
[12] The electrochemical reaction apparatus according to any one of the above [8] to [11],
   wherein an inside of the first reaction section is isolated into a cathode side region and an anode side region by the isolating layer, and
   the electrolytic solution has a convection flow that circulates so that the convection flow exits from the cathode side region to an outside of the first reaction section, and then returns to the cathode side region.
[13] The electrochemical reaction apparatus according to any one of the above [8] to [12],
   wherein an inside of the first reaction section is isolated into a cathode side region and an anode side region by the isolating layer, and
   the electrolytic solution has a convection flow that circulates between the anode side region and the second reaction section.
[14] The electrochemical reaction apparatus according to the above [13], wherein the electrolytic solution further has a convection flow allowing a gas in the electrolytic solution to flow from the cathode side region to a path through which the circulating convection flow flows, so that the gas is to supplied to the second reaction section.
[15] The electrochemical reaction apparatus according to any one of the above [8] to [14],
   wherein the second reaction section comprises a catalyst layer that comprises the second catalyst, and
   the electrolytic solution has a convection flow that flows from below to above in the second reaction section.
[16] The electrochemical reaction apparatus according to the above [15], comprising a gas diffusing mechanism on an underside of the catalyst layer.
[17] The electrochemical reaction apparatus according to any one of the above [8] to [16],
   wherein the second reaction section comprises a catalyst layer that comprises the second catalyst, and
   the second catalyst is an active particle-containing catalyst comprising a support and active particles that are supported on the support and contain a metal element, and
   a metal amount of the active particles in the catalyst layer is 0.1 parts by mass or more and 15 parts by mass or less with respect to 100 parts by mass of the support.
[18] The electrochemical reaction apparatus according to any one of the above [1] to [17], wherein the second catalyst is contained in the electrolytic solution.
[19] The electrochemical reaction apparatus according to any one of the above [1] to [18], wherein the first reaction section is a one-compartment type electrochemical cell.
[20] The electrochemical reaction apparatus according to any one of the above [1] to [19], wherein the first catalyst comprises CoO and a component derived from a pyridine derivative.
[21] The electrochemical reaction apparatus according to any one of the above [1] to [20], wherein the second catalyst comprises at least one of Pd and Au.
[22] The electrochemical reaction apparatus according to any one of the above [1] to [21], wherein the reactant is an alcohol-based compound.
[23] The electrochemical reaction apparatus according to any one of the above [1] to [22], wherein the second catalyst is a catalyst that synthesizes a carbonyl compound from the carbon monoxide and the reactant.
[24] A method of producing an oxygen-containing organic material in which an oxygen-containing organic material is synthesized from carbon dioxide in an electrochemical reaction apparatus comprising: a cathode comprising a first catalyst that reduces carbon dioxide to carbon monoxide; an anode; an electrolytic solution comprising a reactant and an electrolyte; and a second catalyst that synthesizes an oxygen-containing organic material from the carbon monoxide and the reactant,
   a convection flow being formed in the electrolytic solution so that the electrolytic solution comes into contact with the first catalyst and the second catalyst in this order.
[25] The method of producing an oxygen-containing organic material according to the above [24],
   wherein the electrochemical reaction apparatus comprises a first reaction section that comprises the cathode and the anode and a second reaction section that comprises the second catalyst, and
   a direction of the convection flow is formed in the electrolytic solution that flows in an order of the first reaction section and the second reaction section.
[26] An electrochemical reaction apparatus comprising:
   an electrochemical cell comprising a cathode that comprises a first catalyst that reduces carbon dioxide to carbon monoxide and an anode;
   a reaction section comprising a second catalyst that synthesizes an oxygen-containing organic material from the carbon monoxide and a reactant; and
   an electrolytic solution comprising the reactant and an electrolyte,
   the electrolytic solution having a direction of a convection flow that flows in an order of the electrochemical cell and the reaction section.
[27] A method of producing an oxygen-containing organic material in which an oxygen-containing organic material is synthesized from carbon dioxide in an electrochemical reaction apparatus that comprises:
   an electrochemical cell comprising a cathode that comprises a first catalyst that reduces carbon dioxide to carbon monoxide and an anode;
   a reaction section comprising a second catalyst that synthesizes an oxygen-containing organic material from the carbon monoxide and a reactant; and
   an electrolytic solution comprising the reactant and an electrolyte,
   a direction of a convection flow being formed in the electrolytic solution that flows in an order of the electrochemical cell and the reaction section.
[28] An electrochemical cell comprising:
   a cathode comprising a first catalyst that reduces carbon dioxide to carbon monoxide;
   an anode;
   an electrolytic solution comprising a reactant and an electrolyte; and
   a second catalyst that synthesizes an oxygen-containing organic material from the carbon monoxide and the reactant,
   the electrolytic solution having a convection flow, a direction of the convection flow formed so that the electrolytic solution comes into contact with the first catalyst and the second catalyst in this order.
[29] A method for producing an oxygen-containing organic material in which an oxygen-containing organic material is synthesized from carbon dioxide in an electrochemical cell that comprises: a cathode that comprises a first catalyst that reduces carbon dioxide to carbon monoxide; an anode; an electrolytic solution that contains a reactant and an electrolyte; and a second catalyst that synthesizes an oxygen-containing organic material from the carbon monoxide and the reactant,
   wherein a convection flow is formed in the electrolytic solution so that electrolytic solution comes into contact with the first catalyst and the second catalyst in this order.

### Advantageous Effects of Invention

According to the present invention, an oxygen-containing organic material, such as a carbonyl compound, can be electrochemically synthesized from carbon dioxide by an apparatus that has a relatively simple structure.

Also, according to one embodiment of the present invention, the amount of production can be increased while keeping power consumption low when obtaining an oxygen-containing organic material, such as a carbonyl compound, from carbon dioxide.

### Brief Description of Drawings

[Fig. 1] Fig. 1 is a schematic diagram showing an electrochemical reaction apparatus according to the first embodiment of the present invention.
[Fig. 2] Fig. 2 is a schematic diagram showing an electrochemical reaction apparatus according to the second embodiment of the present invention.
[Fig. 3] Fig. 3 is a schematic diagram showing an electrochemical reaction apparatus according to the third embodiment of the present invention.
[Fig. 4] Fig. 4 is a schematic diagram showing an electrochemical reaction apparatus according to a modified example of the third embodiment of the present invention.
[Fig. 5] Fig. 5 is a schematic diagram showing an electrochemical reaction apparatus according to the fourth embodiment of the present invention.
[Fig. 6] Fig. 6 is a schematic diagram showing an electrochemical reaction apparatus according to the fifth embodiment of the present invention.
[Fig. 7] Fig. 7 is a schematic diagram showing a modified example of the electrode arrangement in the fifth embodiment of the present invention.
[Fig. 8] Fig. 8 is a schematic diagram showing an electrochemical reaction apparatus according to the sixth embodiment of the present invention.
[Fig. 9] Fig. 9 is a schematic diagram showing an electrochemical reaction apparatus according to the seventh embodiment of the present invention.
[Fig. 10] Fig. 10 is a schematic diagram showing an electrochemical reaction apparatus according to the eighth embodiment of the present invention.
[Fig. 11] Fig. 11 is a schematic diagram showing an electrochemical reaction apparatus according to the ninth embodiment of the present invention.
[Fig. 12] Fig. 12 is a schematic diagram showing an electrochemical reaction apparatus according to the tenth embodiment of the present invention.
[Fig. 13] Fig. 13 is a schematic diagram showing an electrochemical reaction apparatus according to the eleventh embodiment of the present invention.
[Fig. 14] Fig. 14 is a schematic diagram showing an electrochemical reaction apparatus according to Comparative Example 1.

### Description of Embodiments

Hereinafter, the electrochemical reaction apparatus and method of producing an oxygen-containing organic material of the present invention will be described using embodiments. Note that, in the following description, the X direction is an arbitrary one direction inside the cell, and the Y direction is the direction perpendicular to the X direction.

### <First embodiment>

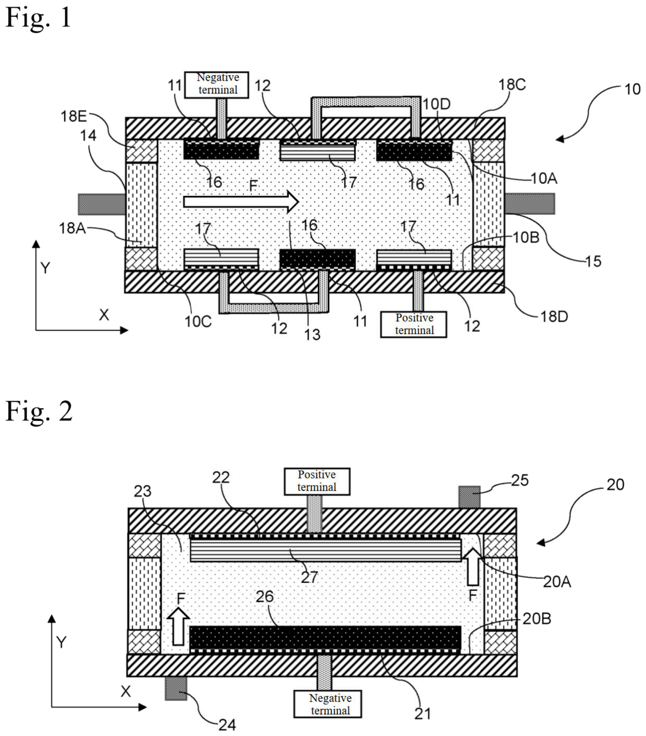

Fig. 1 shows an electrochemical reaction apparatus according to the first embodiment of the present invention. An electrochemical reaction apparatus 10 according to the first embodiment of the present invention is composed of an electrochemical cell comprising cathodes 11, anodes 12, and an electrolytic solution 13 that contains a reactant and an electrolyte. In the present embodiment, the electrochemical reaction apparatus is composed of a single electrochemical cell, and a series of reactions to obtain an oxygen-containing organic material from carbon dioxide are all carried out in the electrochemical cell.

The electrochemical reaction apparatus (electrochemical cell) 10 is provided with a plurality of cathodes 11 and a plurality of anodes 12. Each cathode 11 has a first catalyst 16 that reduces carbon dioxide to carbon monoxide. Each anode 12 has a second catalyst 17 that synthesizes an oxygen-containing organic material from carbon monoxide and a reactant. The second catalyst 17 is preferably a catalyst that synthesizes a carbonyl compound from carbon monoxide and the reactant. The reactant may be anything as long as it is capable of reacting with carbon monoxide to produce an oxygen-containing organic material, but it is preferably a raw material that can produce a carbonyl compound, and more preferably an alcohol-based compound, as will be described later.

In the present embodiment, the electrochemical reaction apparatus 10 is composed of one reaction chamber. The reaction chamber is filled with the electrolytic solution 13. The reaction chamber has wall surfaces 10A and 10B that face each other in the Y direction. In the present embodiment, a plurality of pairs of cathode 11 and anode 12 are provided, and the cathode 11 and the anode 12 in each pair are attached to either of the wall surfaces 10A and 10B separately, so that they face each other in the Y direction. Also, on each of the wall surfaces 10A and 10B, the cathode(s) 11 and the anode(s) 12 are lined up alternately in the X direction.

With the above configuration, the cathode 11 and the anode 12 in the pair that face each other are arranged, with the cathode(s) 11 and the anode(s) 12 being arranged alternately along the X direction. Also, on each of the wall surfaces 10A and 10B of the cell, the first catalysts 16 possessed by the cathode(s) 11 and the second catalysts 17 possessed by the anode(s) 12 are arranged on an identical plane. Note that, in each of the wall surfaces 10A and 10B, the identical plane on which the first and second catalysts 16 and 17 are arranged is a plane parallel to the X direction.

Between the cathode 11 and the anode 12 that face each other in the pair, a voltage is applied by a power source not shown in the figure. Note that, in Fig. 1, the plurality of cathodes 11 and anodes 12 are connected in series and one cathode 11 is connected to a negative terminal and one anode 12 is connected to a positive terminal, but the connection method is not particularly limited. Also, the cathodes 11 and the anodes 12 may be connected to terminal or the like via a current collecting rod or the like, although there is no particular limitation.

On wall surfaces 10C and 10D that face each other in the X direction of the reaction chamber, a supply port 14 and a discharge port 15 are provided, respectively. As a result, the supply port 14 and the discharge port 15 are arranged outside of the anodes 22 and the cathodes 21, so that they interpose the anodes 22 and the cathodes 21 therebetween when viewed along the Y direction.

Note that, in Fig. 1, an aspect is shown in which three pairs of cathode 11 and anode 12 are provided, but any number of pairs of cathode 11 and anode 12 may be provided as long as there are two or more pairs, and for example, four or more pairs may be provided. Also, the reaction chamber may be formed of glass, resin material, or a combination thereof, although there is no particular limitation. The reaction chamber may be assembled by, for example, overlaying a frame member 18A that has the supply port 14 and the discharge port 15, and base plates 18C and 18D that sandwich the frame member 18A, via packings 18E or the like, although there is no particular limitation.

In the present embodiment, the electrolytic solution 13 has a convection flow F as it is allowed to flow from the supply port 14 toward the discharge port 15 and is discharged from the discharge port 15. The electrolytic solution 13 that has been discharged from the discharge port 15 may be circulated and resupplied from the supply port 14, and the circulation may be repeated. Also, the electrolytic solution 13 may be supplied by a pump such as a diaphragm pump, a syringe pump, or a peristaltic pump, or by any other known means other than pumps; or the electrolytic solution 13 may be supplied by utilizing gravity to form the convection flow F, although there is no particular limitation.

The electrolytic solution 13 to be supplied from the supply port 14 may contain carbon dioxide, and the electrolytic solution 13 with which the reaction chamber is filled may also contain carbon dioxide. Note that, although there is no particular limitation on the method for making the electrolytic solution 13 contain carbon dioxide, carbon dioxide may be dissolved in the electrolytic solution 13 by bubbling or other methods before supplying the electrolytic solution 13. It is also preferable that a part of carbon dioxide is dissolved in the electrolytic solution 13 and a part thereof is mixed with the electrolytic solution 13 in the form of bubbles. In addition, the electrolytic solution 13 is sometimes circulated in the electrochemical reaction apparatus 10, as will be described later, and carbon dioxide may be additionally added to the electrolytic solution 13 by bubbling or other methods during the circulation.

By allowing the electrolytic solution 13 to flow from the supply port 14 to the discharge port 15, the convection flow F is formed inside the cell 10 so as to be oriented in the X direction. In other words, the convection flow F is formed so that the electrolytic solution 13 flows in a direction that crosses the direction in which the electrode cells face each other. Therefore, inside the electrochemical reaction apparatus 10, the electrolytic solution 13 comes into contact with the first catalyst 16 possessed by the cathode 11 and the second catalyst 17 possessed by the anode 12, which constitute a pair of electrodes different from each other, in this order. Then, at the cathode 11, the reduction of carbon dioxide is catalyzed by the first catalyst 16 to produce a reduced product of carbon dioxide. The reduced product is carbon monoxide, and the electrochemical reaction that is carried out at the cathode is typically as in the following formula (A).

CO₂ + 2H⁺ + 2e⁻ → CO + H₂O (A)

Also, at the anode 12, an oxygen-containing organic material is produced from the reduced product (carbon monoxide) produced at the cathode 11 and the reactant by the second catalyst 17. The oxygen-containing organic material to be produced is preferably a carbonyl compound. Examples of the carbonyl compound include an organic carbonate, an organic oxalate, a urea-based compound, and a carbonyl halide.

As described above, in the present embodiment, the electrochemical reaction apparatus 10 can synthesize an oxygen-containing organic material, such as a carbonyl compound, using carbon dioxide as a raw material, despite its simple configuration. Also, since all the carbon dioxide contained in the electrolytic solution 13 generally does not react after only single passing through the reaction chamber, the production rate of an oxygen-containing organic material, such as a carbonyl compound, can be enhanced by circulating the electrolytic solution 13.

Furthermore, carbon dioxide may inhibit the synthesis of an oxygen-containing organic material, such as a carbonyl compound and may also generate a side reaction, when it comes into contact with the second catalyst 17. Similarly, an oxidized product or other generated at the anode 12 may inhibit the reduction by the first catalyst 16 or may generate a side reaction, when it comes into contact with the first catalyst 16. However, in the present embodiment, the direction of the convection flow F is formed so that the electrolytic solution 13 comes into contact with the first catalyst 16 and the second catalyst 17 in this order, which decreases the contact of carbon dioxide with the second catalyst 17 and also decreases the contact of the oxidized species generated at the anode 12 with the first catalyst 16. Accordingly, the inhibition of the synthesis of an oxygen-containing organic material, such as a carbonyl compound, or of the reduction of carbon dioxide, or the generation of a side reaction can be decreased, and therefore, an oxygen-containing organic material, such as a carbonyl compound, can be efficiently produced.

Also, in the present embodiment, the first and second catalysts 16 and 17 are arranged on an identical plane that is parallel to the X direction, as described above, and therefore, the convection flow F flowing along the X direction is parallel to the above identical plane. Accordingly, the electrolytic solution 13 efficiently comes into sequential contact with the first and second catalysts 16 and 17, and therefore, the production rate of an oxygen-containing organic material, such as a carbonyl compound, can be enhanced.

Hereinafter, each constituent element to be used in the present embodiment will be described in more detail.

### [Cathode]

The cathode 11 is arranged inside the electrochemical reaction apparatus 10, and includes the first catalyst 16, as described above. The cathode 11 is an electrode used when electrochemically synthesizing a reduced product, such as carbon monoxide, from carbon dioxide.

### (First catalyst)

The first catalyst is a reducing catalyst that is capable of reducing carbon dioxide to a reduced product, such as carbon monoxide. Although there is no particular limitation on the first catalyst as long as it is a catalyst that is capable of reducing carbon dioxide to a reduced product, such as carbon monoxide, it may include, for example, a metal element. The metal element may be a metal itself, or may be a metal compound or the like.

Although there is no particular limitation on the metal element in the above metal, examples thereof include V, Cr, Mn, Fe, Co, Ni, Cu, Sn, Zr, Nb, Mo, Ru, Rh, Pd, Ag, Cd, Hf, Ta, W, Re, Ir, Pt, Au, Hg, Al, Si, In, Sn, Tl, Pb, Bi, Sb, Te, U, Sm, Tb, La, Ce, Zn, Os, and Nd.

As the above metal compound, metal compounds such as inorganic metal compounds and organic metal compounds of these metals can be used, and specific examples thereof include a metal halide, a metal oxide, a metal hydroxide, a metal nitrate, a metal sulfate, a metal acetate, a metal phosphate, a metal carbonyl, and a metal acetylacetonate.

The metal element to be used in the first catalyst is preferably a metal element from Group 7 to Group 12. Preferable specific examples thereof include Mn, Fe, Ni, Ru, Co, Rh, Cu, Zn, Ag, Au, Pd, Ir, Pt, and Os, among which Co, Fe, Ni, Au, and Ag are more preferable, and Co is particularly preferable. The use of these metal elements makes it easier to increase the conversion efficiency of carbon dioxide to carbon monoxide, resulting in higher catalytic activity. One type of metal may be used alone, or two or more types thereof may be used in combination in the metal derivative.

The first catalyst may contain a carbon compound in addition to the above metal or metal compound. As the carbon compound, a conductive carbon compound is preferable. More specific examples of the carbon compound include mesoporous carbon, activated carbon, carbon black such as ketjen black and acetylene black, graphite, carbon fiber, graphene, and carbon nanotube, among which carbon black is preferable, and as for the carbon black, conductive carbon black is still more preferable. The carbon compound is also preferably porous carbon.

In the case where the first catalyst contains a carbon compound, the metal or metal compound may be supported on the carbon compound. Also, the carbon compound may be made into a catalyst powder or the like by mixing it with a complex or the like that includes the above metal element and then subjecting the mixture to a heat treatment. The first catalyst in which the metal or metal compound is supported on the carbon compound may be further supported on an electrode substrate, which will be described later.

### <<Nitrogen-containing metal catalyst>>

The first catalyst is also preferably a catalyst that contains nitrogen element and a metal element (also referred to as "nitrogen-containing metal catalyst"). Here, the nitrogen element to be used in the nitrogen-containing metal catalyst is preferably derived from a nitrogen-containing compound, which will be described later. Also, specific examples of the metal element to be used in the nitrogen-containing metal catalyst are as described above, and preferable metal elements thereof are also as described above. The use of the nitrogen-containing metal catalyst increases the production efficiency of carbon monoxide, and as a result, the selectivity for the oxygen-containing organic material to be synthesized, such as a carbonyl compound, is also increased.

The nitrogen-containing metal catalyst may be a catalyst formed by subjecting a mixture containing a metal derivative and a nitrogen-containing compound (hereinafter, referred to as "first catalyst raw material mixture") to a heat treatment, and among the above, it is preferably a catalyst formed by subjecting a first catalyst raw material mixture containing a metal derivative, a nitrogen-containing compound, and a carbon compound to a heat treatment. Note that the heat treatment is typically sintering.

By subjecting the first catalyst raw material mixture to a heat treatment to produce the nitrogen-containing metal catalyst, metal-nitrogen element bonds derived from the metal derivative and nitrogen-containing compound may be formed in the catalyst. Also, the carbon compound functions as a support in the catalyst, and the components derived from the metal derivative and nitrogen-containing compound are supported on the carbon compound. The nitrogen-containing metal catalyst having the above configuration efficiently reduces carbon dioxide to carbon monoxide and its conversion efficiency is high.

The metal element in the metal derivative is as described above. The metal derivative preferably includes metal ions. Also, the metal derivative may be used in the form of a metal salt, for example. Examples of the metal salt include a metal nitrate, a metal sulfate, a metal chloride, a metal bromide, a metal iodide, and a metal acetate, among which a metal nitrate is preferable. Specifically, cobalt nitrate (Co(NO₃)₂), nickel nitrate (Ni(NO₃)₂), iron nitrate (Fe(NO₃)₂), manganese nitrate (Mn(NO₃)₂), copper nitrate (Cu(NO₃)₂), and zinc nitrate (Zn(NO₃)₂) are preferable. One type of metal salt may be used alone, or two or more types thereof may be used in combination.

The metal content derived from the above metal derivative in the first catalyst raw material mixture is, for example, 0.1% by mass or more and 50% by mass or less, but it is preferably 0.1% by mass or more and 10% by mass or less, with respect to the entire amount of the first catalyst raw material mixture. Within the above range, the metal is contained in the catalyst without aggregation, and an appropriate amount of catalytic active sites are formed. Therefore, it is easier to increase the conversion efficiency to carbon monoxide.

From the viewpoints of conversion efficiency and other factors, the above metal content is more preferably 0.5% by mass or more, still more preferably 1.0% by mass or more, and even more preferably 1.5% by mass or more, and it is also more preferably 10% by mass or less, and still more preferably 5% by mass or less. Note that the entire amount of the first catalyst raw material mixture means the entire amount of the solid content of the first catalyst raw material mixture, and in the case where a volatile component is blended into the first catalyst raw material mixture during the production process, it is the amount excluding that volatile component.

### (Nitrogen-containing compound)

The nitrogen-containing compound is a compound that contains nitrogen, and a component derived from the nitrogen-containing compound may form bonds, such as coordinate bonds, with the metal element derived from the metal derivative in the nitrogen-containing metal catalyst. Specific examples of the nitrogen-containing compound include a compound that includes a nitrogen-containing aromatic ring that has nitrogen element as a constituent element of the aromatic ring. Specific examples of the nitrogen-containing compound include a pyridine derivative, an imidazole derivative, a pyrazole derivative, and a triazole derivative. One of these compounds may be used alone, or two or more thereof may be used in combination.

Among these, from the viewpoints of conversion efficiency and other factors, the nitrogen-containing compound is preferably any selected from the group consisting of a pyridine derivative, an imidazole derivative, and a triazole derivative, with a pyridine derivative being particularly preferable. In other words, the nitrogen-containing metal catalyst is particularly preferably a catalyst obtained by subjecting a mixture including a metal derivative, a pyridine derivative, and a carbon compound to a heat treatment.

### (Pyridine derivative)

The pyridine derivative is a compound that has a pyridine ring. The pyridine derivative may be a compound that has one, may be a compound that has two, may be a compound that has three, or may be a compound that has four or more pyridine rings in one molecule.

The pyridine derivative may be 4-aminopyridine, 2,2'-bipyridine, 4,4'-diamino-2,2'-bipyridine, or the like, and examples thereof also include a polymer that has a plurality of pyridine rings in one molecule and has a weight-average molecular weight of 1,000 or more. As specific compounds of the polymer, polypyridine such as poly(2,5-pyridine) and poly(3,5-pyridine), and polyvinylpyridine, a polymer of vinylpyridine, are preferable, among which poly(2,5-pyridine) and poly(4-vinylpyridine) are more preferable, and poly(4-vinylpyridine) is still more preferable. Polyvinylpyridine such as poly(4-vinylpyridine) preferably has a molecular weight at or above a certain level from the viewpoints of conversion efficiency and other factors, and the weight-average molecular weight is, for example, 1,000 or more, 10,000 or more, preferably 30,000 or more, and still more preferably 50,000 or more. Also, the weight-average molecular weight is, for example, 200,000 or less, and preferably 100,000 or less, from the viewpoint of ease of availability and the like.

Note that the weight-average molecular weight is a value measured by gel permeation chromatography (GPC), and polystyrene may be used as a reference material.

The amount of the nitrogen-containing compound blended in the first catalyst raw material mixture is preferably adjusted so that the molar ratio of the nitrogen-containing aromatic ring of the nitrogen-containing compound with respect to the metal element of the above metal derivative (nitrogen-containing aromatic ring/metal element) is 2 or more and 20 or less. Within the above range, the generation of a side reaction can be suppressed and a catalyst that contains an appropriate amount of metal-nitrogen element bonds can be produced, making it easier to increase the conversion efficiency or the like described above. From the viewpoints of conversion efficiency and other factors, the above molar ratio is more preferably 2 or more, and still more preferably 4 or more, and it is also more preferably 18 or less, and more preferably 15 or less. Note that the above molar ratio represents the ratio between the number of nitrogen-containing aromatic rings included in the nitrogen-containing compound and the number of moles of the metal element included in the metal derivative.

There is no particular limitation on the carbon compound to be used in the nitrogen-containing metal catalyst as long as it can support a heating-treated product (for example, sintered product) of the metal derivative and the nitrogen-containing compound such as a pyridine derivative, but a conductive carbon compound is preferable. The use of the conductive carbon compound increases the electrical conductivity at the cathode and makes it easier to increase the conversion efficiency to carbon monoxide. Specific examples of the carbon compound are as described above, with carbon black being preferable, and in particular, conductive carbon black is still more preferable.

Although there is no particular limitation on the content of the carbon compound in the first catalyst raw material mixture with respect to the entire amount of the first catalyst raw material mixture, it is, for example, 5% by mass or more and 90% by mass or less, preferably 15% by mass or more and 80% by mass or less, and more preferably 20% by mass or more and 70% by mass or less. By keeping the content of the carbon compound within the above range, the heating-treated product of the metal derivative and the nitrogen-containing compound can be appropriately supported while maintaining good catalytic activity.

The nitrogen-containing metal catalyst may be in powder or particle form. When the nitrogen-containing metal catalyst is in powder or particle form, it is easier to make it supported on an electrode substrate, which will be described later. Also, the contact area to carbon dioxide tends to be larger, which makes it easier to improve the conversion efficiency to carbon monoxide.

The nitrogen-containing metal catalyst obtained by the heat treatment has the metal element and nitrogen element as described above, but the metal element is preferably contained in the nitrogen-containing metal catalyst as a metal oxide from the viewpoints of selectivity, ease of production, and other factors. The metal element is preferably cobalt, as described above, and accordingly, the nitrogen-containing metal catalyst preferably contains cobalt oxide. Also, the cobalt oxide particularly preferably contains CoO.

In addition, as described above, the nitrogen-containing metal catalyst is preferably subjected to a heat treatment to have a component derived from a nitrogen-containing compound, and in particular, it preferably has a component derived from a pyridine derivative. Accordingly, it is particularly preferable for the nitrogen-containing metal catalyst to have CoO and the component derived from a pyridine derivative. Then, nitrogen atoms derived from the nitrogen-containing compound may coordinate to the metal element, such as cobalt element.

The component derived from the nitrogen-containing compound preferably includes a nitrogen-containing aromatic ring structure, and specific examples thereof include a pyridine ring structure, an imidazole ring structure, a pyrazole ring structure, and a triazole ring structure. Among these, a pyridine ring structure, an imidazole ring structure, and a triazole ring structure are preferable, and a pyridine ring structure is particularly preferable. The coordination of nitrogen in the pyridine ring structure to cobalt is presumed to lower the activation energy of the reaction intermediate, thereby increasing the conversion efficiency or the like.

Note that, as described above, the nitrogen-containing metal catalyst is produced by subjecting a mixture that includes a nitrogen-containing compound to a heat treatment or the like, but the treatment temperature is low. Accordingly, the nitrogen-containing aromatic ring contained in the nitrogen-containing compound can remain in the nitrogen-containing metal catalyst.

It can be confirmed by X-ray photoelectron spectroscopy (XPS) or the like that the nitrogen-containing metal catalyst has a nitrogen-containing aromatic ring structure such as the pyridine ring structure. In addition, it can be also confirmed by X-ray photoelectron spectroscopy (XPS) that nitrogen element is coordinated to the metal element such as cobalt element. Furthermore, the valence of the metal element, for example, that the cobalt oxide is divalent, can be confirmed by XPS, X-ray diffraction measurement (XRD) or the like.

In addition to the above metal oxide, the nitrogen-containing metal catalyst may also contain various metal elements as metals. Accordingly, in the case where the metal element is cobalt, the nitrogen-containing metal catalyst may contain cobalt metal in addition to the cobalt oxide. The cobalt metal may have crystallinity, for example. In the case where the nitrogen-containing metal catalyst contains cobalt metal, it preferably has a core-shell structure in which the cobalt metal is the core and the cobalt oxide is arranged around the core so as to cover that cobalt metal. When the nitrogen-containing metal catalyst has a core-shell structure, it is presumed that the conductivity of the nitrogen-containing metal catalyst is improved, making it easier to improve the reduction rate for carbon dioxide or the like. Note that the core-shell structure may be in particle form to be supported on the carbon compound.

### <<Method for producing nitrogen-containing metal catalyst>>

The nitrogen-containing metal catalyst can be produced by subjecting the first catalyst raw material mixture that includes a metal derivative and a nitrogen-containing compound, such as a pyridine derivative, or a metal derivative, a nitrogen-containing compound, and a carbon compound, to a heat treatment. At this time, the first catalyst raw material mixture is preferably subjected to a heat treatment by heating to a heat treatment temperature of 150°C or higher and 550°C or lower. The heat treatment temperature is more preferably 180°C or higher and 500°C or lower, and still more preferably 200°C or higher and 470°C or lower. The above heat treatment is preferably carried out in an inert gas atmosphere such as argon or nitrogen gas.

The first catalyst raw material mixture can be subjected to a heat treatment at the heat treatment temperature described above to form metal-nitrogen element bonds, and the components derived from the metal derivative and nitrogen-containing compound may be supported on the carbon compound. Although there is no particular limitation on the time for which the first catalyst raw material mixture is subjected to a heat treatment (heat treatment time), it is, for example, 0.5 hours or more and 10 hours or less, preferably 1 hour or more and 8 hours or less, and more preferably 2 hours or more and 5 hours or less.

The first catalyst raw material mixture to be subjected to a heat treatment is preferably in powder or particle form. By being in powder or particle form, a catalyst in powder or particle form can be obtained by the heat treatment. Also, the first catalyst raw material mixture to be subjected to a heat treatment more preferably consists of a metal derivative, a nitrogen-containing compound, and a carbon compound.

The first catalyst raw material mixture may be obtained by, for example, preparing a dilute solution of the first catalyst raw material mixture in which a metal derivative and a nitrogen-containing compound, or a metal derivative, a nitrogen-containing compound, and a carbon compound have been diluted with a dilution solvent, and drying that dilute solution. The first catalyst raw material mixture may be obtained in powder or particle form, for example.

Also, in the dilute solution of the first catalyst raw material mixture, each component (metal derivative, nitrogen-containing compound, and carbon compound) is preferably dispersed or dissolved in the dilution solvent. By dispersing or dissolving each component in the dilution solvent, a first catalyst raw material mixture in which each component has been homogeneously mixed can be obtained.

Although there is no particular limitation on the method for obtaining the above dilute solution of the first catalyst raw material mixture, it may be obtained by, for example, first adding a nitrogen-containing compound to a dispersion medium and dispersing it, and then further adding a metal derivative and, if necessary, a carbon compound to that dispersion.

As the dilution solvent to be used to dilute the first catalyst raw material mixture, water or an organic solvent can be used, but an organic solvent is preferable. Examples of the organic solvent include, but are not particularly limited to, an ester-based solvent, a ketone-based solvent, an ether-based solvent, an alcohol-based solvent, a glycol ether, an amide-based solvent, a nitrile-based solvent, a carbonate-based solvent, a halogenated hydrocarbon, a hydrocarbon, a sulfone-based solvent, a sulfoxide, and formamide. As the dilution solvent, a mixed solvent of an organic solvent and water may also be used.

Although there is no particular limitation on the concentration of the dilute solution of the first catalyst raw material mixture, it is, for example, 0.01 to 50 g/L, and preferably 1 to 10 g/L.

In addition to the above, the first catalyst may be a carbon compound that contains at least one of a heteroatom such as nitrogen or a metal or metal compound. Examples of such a carbon compound include nitrogen-containing graphite, nitrogen-containing carbon nanotube, nitrogen-containing graphene, Ni and nitrogen-containing graphite, Ni and nitrogen-containing carbon nanotube, Ni and nitrogen-containing graphene, Cu and nitrogen-containing graphite, Cu and nitrogen-containing carbon nanotube, Cu and nitrogen-containing graphene, Co and nitrogen-containing graphite, Co and nitrogen-containing carbon nanotube, and Co and nitrogen-containing graphene.

In the electrochemical reaction apparatus 10, one type of first catalyst may be used alone, or two or more types thereof may be used in combination.

### (Electrode substrate)

The cathode 11 may have an electrode substrate (current collector). There is no particular limitation on the electrode substrate as long as it is a current collector that is conventionally used in a carbon dioxide reduction electrode. Examples thereof include a carbon substrate, a metal substrate, and a metal oxide substrate, and it preferably has conductivity. Also, the electrode substrate may be a porous material. The substrate is a substrate that constitutes the electrode, and may be in the form of a sheet, plate, or the like, or it may be in the form of a layer laminated on the wall surface or the like of the reaction chamber.

Examples of the carbon substrate include porous carbon, such as carbon non-woven fabric. There is no particular limitation on the carbon non-woven fabric, and any known carbon non-woven fabric can be used. For example, those commercially available as carbon non-woven fabrics for fuel cells can be used, and examples thereof include "TORAYCA" (R) carbon paper and Toray 060 manufactured by Toray Industries, Inc., "AvCarb 1071 HCB" manufactured by New Metals and Chemicals Corporation, Ltd., and the BC series manufactured by SGL Carbon AG.

Also, examples of the metal substrate include metal mesh, and examples of the metal to be used include gold, silver, platinum, nickel, titanium, and chromium. In addition, examples of the metal oxide to be used in the metal oxide substrate include indium oxide, tin oxide, tin-doped indium oxide, and fluorine-doped tin oxide (FTO).

The first catalyst may be supported on the electrode substrate. There is no particular limitation on the method for supporting the first catalyst on the electrode substrate. The first catalyst may be attached to the electrode substrate, and in particular, it is preferable to attach the nitrogen-containing metal catalyst described above to the electrode substrate. Note that adhesion here refers to an aspect in which the catalyst is physically fixed to the electrode substrate, and the atoms that constitute the electrode substrate are not chemically bonded to the atoms that constitute the catalyst. Accordingly, even in the case where the electrode substrate includes a carbon compound, such as porous carbon, that carbon compound itself does not have the metal-nitrogen element bonds described above. It does not have metal-carbon element bonds either. Note that carbon element here refers to carbon element that constitutes the carbon compound, such as porous carbon, and metal is metal element derived from the metal derivative. The nitrogen-containing metal catalyst is attached to the electrode substrate without being chemically bonded, which makes it easier to enhance the conversion efficiency to carbon monoxide and also makes its production easier.

However, the atoms that constitute the first catalyst may be chemically bonded to the atoms that constitute the electrode substrate. For example, in the case where the electrode substrate includes a carbon compound, such as porous carbon, that carbon compound itself may have the metal-nitrogen element bonds and metal-carbon element bonds described above.

Also, the first catalyst may be supported on the electrode substrate together with a catalyst additive and the like. The catalyst additive also functions as a binding agent when the catalyst is supported on the electrode substrate. Furthermore, it also functions as an ion conductor and improves the electrochemical reaction efficiency.

Examples of the catalyst additive include a cation conductive compound, an anion conductive compound, and a fluorine compound other than cation conductive compounds and anion conductive compounds.

Examples of the cation conductive compound include a compound having a functional group or the like that has a Bronsted acid or salt thereof and is anionized by the release of a hydrogen ion or the like. Examples of the cation conductive compound include a compound that has at least one functional group such as a sulfonyl group, a phosphate group, a hydroxyl group, and a silicate group. Specifically, examples of commercially available products include "Nafion" (TM of DuPont de Nemours, Inc.).

Examples of the catalyst additive also include an anion conductive compound. Examples of the anion conductive compound include a compound having a functional group or the like that has a Bronsted base or salt thereof, and for example, it is a compound having a functional group that is cationized by the addition of a proton. The anion conductive compound is excellent in conductivity of anions, such as hydroxide ions.

Specific examples of the anion conductive compound include a compound that has a functional group such as a pyridinium group, an imidazolium group, an amino group, and an ammonium group. As the anion conductive compound, commercially available products such as "Fumion FAA-3-SOLUT-10" from FuMA-Tech GmbH and "PowerMax NXS125 OH" from Resintech can also be used.

Also, examples of the fluorine compound other than cation conductive compounds and anion conductive compounds include polytetrafluoroethylene (PTFE), tetrafluoroethylene oligomer (TFEO), graphite fluoride ((CF)n), and pitch fluoride (FP).

The catalyst additive may have a shape in powder or particle form, for example. In the case where a catalyst additive is used, the first catalyst may be supported on the electrode substrate in a form of a mixture (catalyst composition) in which it is mixed with the catalyst additive. In the case where the catalyst is supported on the electrode substrate together with a catalyst additive, the content of the catalyst additive with respect to the total amount of the catalyst additive and the catalyst is preferably 10% by mass or more and 50% by mass or less, more preferably 15% by mass or more and 45% by mass or less, and still more preferably 20% by mass or more and 40% by mass or less, from the viewpoint of increasing the conversion efficiency of the catalyst and the like.

Although there is no particular limitation on the method for supporting the first catalyst on the electrode substrate, examples thereof include a method in which a dilute solution in which the first catalyst and a component other than the catalyst, such as a catalyst additive, added if necessary, have been diluted with a dilution solvent is applied to the electrode substrate by using various coating apparatuses or by spray coating and then dried, and a method in which the electrode substrate is immersed in the above dilute solution and then dried.

In the cathode, the first catalyst (or catalyst composition) may be formed in a layered shape on the surface of the electrode substrate as a catalyst layer by applying a coating solution that includes the catalyst, or by other methods. Also, the catalyst layer may be formed in a layered shape so that a part or all of the electrode substrate in the thickness direction is impregnated inside with part or all of the catalyst layer, and for example, it may be formed in the layered shape from the surface to the inside of the electrode substrate. Note that the catalyst layer may have voids as appropriate.

Also, in the cathode, the catalyst is supported on one surface and the catalyst layer is formed thereon, but no catalyst may be supported on the other surface and no catalyst layer may be formed on it, or the catalyst may be supported on both surfaces and the catalyst layer may be formed on both surfaces.

Also, the above dilute solution is preferably a dispersion in which the catalyst, or the catalyst and a component other than the catalyst, has been dispersed in a dilution solvent, and water or an organic solvent may be used as the dilution solvent. Although the organic solvent is as described above, preferably, an alcohol-based solvent, a ketone-based solvent, a nitrile-based solvent, or the like may be used. The above dilute solution may be a mixture of an organic solvent and water.

### [Anode]

The anode 12 is arranged inside the electrochemical reaction apparatus 10, and in the present embodiment, includes the second catalyst 17, as described above. In the present embodiment, the anode 12 is an electrode which is used when electrochemically synthesizing an oxygen-containing organic material, such as a carbonyl compound, from the reduced product (carbon monoxide).

### (Second catalyst)

Although there is no particular limitation on the second catalyst as long as it catalyzes the electrochemical reaction that synthesizes an oxygen-containing organic material, such as a carbonyl compound, from the carbon monoxide and the reactant, it may include a metal element, and in particular, it preferably includes a metal element selected from the group consisting of Group 8 to Group 11 elements. The use of Group 8 to Group 11 elements in the catalyst makes it easier to electrochemically synthesize a carbonyl compound, in particular an organic carbonate, from the carbon monoxide at a high selectivity.

Examples of the carbonyl compound include an organic carbonate, an organic oxalate, a urea-based compound, and a carbonyl halide. Among these, at least any of an organic carbonate and an organic oxalate is preferable, and an organic carbonate is more preferable. Accordingly, the second catalyst is preferably a catalyst that catalyzes the reaction that synthesizes an organic carbonate, an organic oxalate, or both of them, and is more preferably a catalyst that catalyzes the reaction that synthesizes at least an organic carbonate.

Specific examples of Group 8 to Group 11 elements to be used in the second catalyst include Fe, Co, Ni, Cu, Ru, Rh, Pd, Ag, Os, Ir, Pt, and Au. Among these, Co, Ni, Cu, Rh, Pd, Ag, Ir, Au, and Pt are preferable, and in particular, Pd, Au, Ag, and Ir are more preferable. The use of each of the elements described above makes it easier to electrochemically synthesize an organic carbonate, an organic oxalate, or both of them from the carbon monoxide at a high selectivity. From these viewpoints, as the metal element contained in the catalyst, Au and Pd are still more preferable, and Pd is particularly preferable.

As for the metal element to be used in the second catalyst, one type of metal element may be used alone, or two or more types thereof may be used in combination. In the case where two or more types are used in combination, two or more metal elements selected from the group consisting of Group 8 to Group 11 elements may be used in combination, or a metal element from Group 8 to Group 11 elements may be used in combination with a metal element other than Group 8 to Group 11 elements. Preferable examples of the metal element other than Group 8 to Group 11 elements include a metal element from period 4 elements, but it may be other than metal elements from period 4 elements.

Specifically, it is preferable to combine Au with at least one selected from the group consisting of Ti, Mn, Fe, Co, Ni, Cu, and Zn. In addition, it is also preferable to combine Pd with at least one selected from the group consisting of Ti, Co, Ni, Cr, Mn, Fe, Cu, Zn, Ru, Rh, Ag, Ir, Pt, Au, and Sn. Furthermore, it is also preferable to combine Ir with at least one selected from the group consisting of Au, Rh, and Ru. These combinations can maintain a high selectivity when synthesizing a carbonyl compound while keeping the amount of precious metal used low.

From the viewpoint of improving selectivity, in the case where two or more types are used in combination, the metal element to be used in the second catalyst preferably includes at least two metal elements selected from the group consisting of Group 8 to Group 11 elements. In particular, it is more preferable to use either Pd or Ir in combination with at least one element selected from the group consisting of Group 8 to Group 11 elements other than either Pd or Ir. Specifically, the combination of Pd with at least one selected from the group consisting of Ag, Au, Pt, Ir, and Cu, or the combination of Ir with one selected from the group consisting of Au, Rh, and Ru, is more preferable.

In the case where two or more metals are used in combination, the content of each metal may be set as appropriate, but in the case where Pd is used, the content of Pd is, for example, 10% by mol or more and 99% by mol or less, and from the viewpoint of selectivity, preferably 25% by mol or more and 95% by mol or less. In this case, the content of metal other than Pd (for example, at least one selected from the group consisting of Ag, Au, Pt, Ir, and Cu) is, for example, 1% by mol or more and 90% by mol or less, and preferably 5% by mol or more and 75% by mol or less.

Also, in the case where Ir is used other than in combination with Pd, the content of Ir is, for example, 5% by mol or more and 99% by mol or less, and from the viewpoint of selectivity, preferably 10% by mol or more and 95% by mol or less, and more preferably 25% by mol or more and 90% by mol or less. In this case, the content of metal other than Ir (for example, at least one selected from the group consisting of Au, Rh, and Ru) is, for example, 1% by mol or more and 95% by mol or less, preferably 5% by mol or more and 90% by mol or less, and more preferably 10% by mol or more and 75% by mol or less.

Note that the content of metal referred to here is a proportion with respect to the entire amount of metal contained in the catalyst.

The second catalyst may be an active particle-containing catalyst, may be a metal salt, or they may be used in combination. The use of an active particle-containing catalyst or a metal salt can produce a carbonyl compound, in particular an organic carbonate, from the carbon monoxide at high conversion efficiency. Among the above, the use of an active particle-containing catalyst is particularly preferable.

### <<Active particle-containing catalyst>>

Active particles in the active particle-containing catalyst have the catalytic ability to catalyze the reaction when electrochemically synthesizing a carbonyl compound from carbon monoxide. There is no particular limitation on the active particles that include a metal element as long as they include a metal element, and they may be constituted by a metal oxide, may be constituted by a metal itself, or may be constituted by both a metal oxide and a metal. The metal element to be used in the active particles is as described above.

In the second catalyst, the active particles are, for example, in the form of fine particles, and although not particularly limited, they are preferably nano-order particles and may have an average particle diameter of preferably 100 nm or less, and more preferably 1 nm or more and 40 nm or less. The active particles have the particle diameter described above to be in nanostructure, which increases the active area and makes it easier to enhance the various performances of the catalyst. Note that the particle diameter means an area equivalent circle diameter, which is obtained by determining the area of each particle in image observation with TEM-EDX or the like, and calculating the diameter from the area assuming that each particle is a circle.

It is also preferable that the active particle-containing catalyst further contains a support, and that the active particles are supported on the support. Although there is no particular limitation on the support to be used in the active particle-containing catalyst, examples thereof include carbon, silica, aluminum oxide, and zirconium oxide. From the viewpoint of being able to synthesize a carbonyl compound from the carbon monoxide at a high selectivity, carbon, silica, and aluminum oxide are preferable. Also, the support is preferably a porous material.

Although there is no particular limitation on the support to be used in the active particle-containing catalyst, from the viewpoint of being able to synthesize a carbonyl compound from the carbon monoxide at a high selectivity, porous carbon is preferable. Accordingly, the active particle-containing catalyst is preferably a catalyst that has active particles having a metal element and porous carbon supporting the active particles. The support being porous carbon makes it easier for the reactant described later, to be diffused appropriately in the catalyst, thereby enhancing the selectivity, reaction efficiency, and the like when synthesizing a carbonyl compound.

It is preferable that the carbon is porous carbon, but similarly, it is preferable that the silica and aluminum oxide are also porous materials.

The active particle-containing catalyst that has a support, such as porous carbon, can be produced by mixing a metal precursor and a support (for example, porous carbon, silica, or aluminum oxide, preferably porous carbon) and subjecting the mixture to a heat treatment, as will be described later. The metal precursor becomes active particles by the heat treatment, and the active particles are supported on the support, such as porous carbon.

Although there is no particular limitation on the porous carbon to be used in the active particle-containing catalyst, it is preferably composed of a carbon compound in powder or particle form, and thus, the active particle-containing catalyst may also be in powder or particle form. When the active particle-containing catalyst is in powder or particle form, it is easier to disperse it in the electrolytic solution and to increase the contact area to carbon monoxide, thereby making it easier to improve the selectivity, reaction efficiency, and the like when synthesizing a carbonyl compound. Supports other than porous carbon are also preferably in powder or particle form.

The BET specific surface area of the porous carbon to be used in the active particle-containing catalysts is, for example, 10 m²/g or more and 3000 m²/g or less, and preferably 100 m²/g or more and 1500 m²/g or less. The BET specific surface area can be measured by gas adsorption analysis.

The average primary particle diameter of the porous carbon to be used in the active particle-containing catalysts is, for example, 1 nm or more and 1000 nm or less, and preferably 10 nm or more and 300 nm or less. The average primary particle diameter of the porous carbon can be measured by observation through electron microscopy for example or the like; specifically, it is exemplified by a method in which by determining the area of each particle and calculating the diameter from the area assuming that each particle is a circle, the area equivalent circle diameter is determined.

The average pore size of the porous carbon is, for example, 0.5 nm or more and 100 nm or less, and preferably 1 nm or more and 50 nm or less. Note that the average pore size of the porous carbon can be measured by gas adsorption analysis.

Although there is no particular limitation on the porous carbon to be used in the active particle-containing catalyst as long as it can support active particles, a conductive carbon compound is preferable. The use of the conductive carbon compound increases the electrical conductivity at the electrode and makes it easier to increase the reaction efficiency and the like.

More specific examples of the porous carbon include mesoporous carbon, activated carbon, carbon black such as ketjen black and acetylene black, carbon nanotube, graphite, and graphene, among which carbon black is preferable, and as for the carbon black, conductive carbon black is still more preferable.

The active particle-containing catalyst may further have a component derived from a nitrogen-containing compound. When the active particle-containing catalyst has a component derived from a nitrogen-containing compound, it is easier to improve the conversion efficiency, selectivity, and the like when synthesizing a carbonyl compound.

The component derived from a nitrogen-containing compound has nitrogen element, and the nitrogen element may coordinate to a metal element that constitutes the active particles (for example, a metal element that constitutes a metal oxide) to form metal-nitrogen element bonds by coordinate bonds.

The component derived from a nitrogen-containing compound may be supported on a support, such as porous carbon, silica, or aluminum oxide, and preferably on porous carbon. In other words, the component derived from a nitrogen-containing compound may coordinate to a metal element that constitutes the active particles and be supported on a support, such as porous carbon. It is preferable that the nitrogen-containing compound has a nitrogen-containing aromatic ring structure in which nitrogen is included in the aromatic ring.

In the case where the active particle-containing catalyst contains a component derived from a nitrogen-containing compound, it can be obtained by subjecting a mixture of a metal precursor, porous carbon, and a nitrogen-containing compound to a heat treatment, as will be described later. Accordingly, the component derived from a nitrogen-containing compound is a component obtained by subjecting the nitrogen-containing compound to a heat treatment. Since the heat treatment temperature is low as will be described later, the nitrogen-containing aromatic ring structure constituted by the nitrogen-containing compound remains in the catalyst. Then, the component derived from a nitrogen-containing compound in the active particle-containing catalyst preferably has a nitrogen-containing aromatic ring structure. Note that the details of the nitrogen-containing compound and nitrogen-containing aromatic ring structure are as described in the first catalyst.

### <<Method for producing active particle-containing catalyst>>

Next, the method for producing the active particle-containing catalyst will be described. The active particle-containing catalyst described above may be obtained by subjecting at least a metal precursor to a heat treatment, but it can be obtained preferably by mixing a metal precursor and a support, such as porous carbon, silica, or aluminum oxide, and more preferably by mixing a metal precursor and porous carbon, and then subjecting the mixture that includes the metal precursor and the support to a heat treatment. Note that the metal precursor or the mixture that includes the metal precursor and porous carbon, which is the raw material for the active particle-containing catalyst, is also hereinafter referred to as second catalyst raw material. The heat treatment is typically sintering. Also, in the case where the active particle-containing catalyst includes a component derived from a nitrogen-containing compound, the nitrogen-containing compound is further mixed with the metal precursor and the porous carbon, and the second catalyst raw material may include the metal precursor, the porous carbon, and the nitrogen-containing compound.

The metal precursor is a compound that becomes the active particles described above by subjecting it to a heat treatment. Accordingly, the metal precursor may be a precursor that has the metal element selected from the group consisting of Group 8 to Group 11 elements described above, and suitable metal elements are also as described above. One type of metal element may be used alone, or two or more types thereof may be used in combination in the metal precursor. The combination of metal elements in the case where two or more types are used in combination is as described above.

Note that, in the case where two or more metal precursors are used in combination, the active particle-containing catalyst may be obtained by mixing two or more metal precursors or by mixing two or more metal precursors and porous carbon and then subjecting the mixture to a heat treatment, and accordingly, two or more metal precursors may be contained in the second catalyst raw material.

The metal precursor preferably includes metal ions. Also, the metal precursor may be used in the form of a metal salt, for example. Examples of the metal salt include a metal nitrate, a metal sulfate, a metal chloride, a metal bromide, a metal iodide, and a metal acetate, among which a metal chloride and a metal nitrate are preferable, and from the viewpoint of being able to form appropriate active particles, a metal nitrate is more preferable.

Note that specific examples of the metal nitrate and metal chloride to be used in the metal precursor are the same as the metal salts to be used as the second catalyst, which will described later, and the description therefor will thus be omitted. Also, the metal salt may be a hydrate.

In addition, the porous carbon and nitrogen-containing compound to be used as raw materials in the present production method are as described in the first catalyst described above.

The metal content derived from the above metal precursor in the second catalyst raw material is preferably 0.1% by mass or more and 70% by mass or less, and still more preferably 2% by mass or more and 50% by mass or less, with respect to the entire amount of the second catalyst raw material. Within the above range, the metal is contained in the catalyst without aggregation, and an appropriate amount of catalytic active sites are formed. Therefore, it is easier to increase the selectivity and the like when synthesizing a carbonyl compound, and the reaction efficiency and the like are also increased.

Also, although there is no particular limitation on the content of the support, such as porous carbon, in the second catalyst raw material with respect to the entire amount of the second catalyst raw material, it is, for example, 10% by mass or more and 95% by mass or less, preferably 20% by mass or more and 85% by mass or less, and more preferably 30% by mass or more and 80% by mass or less. By keeping the content of the support, such as porous carbon, within the above range, the active particles and the component derived from a nitrogen-containing compound can be appropriately supported while maintaining good catalytic activity.

In the case where a nitrogen-containing compound is used, the amount of the nitrogen-containing compound blended in the second catalyst raw material is preferably adjusted so that the molar ratio of the nitrogen-containing aromatic ring of the nitrogen-containing compound with respect to the metal element derived from the above metal precursor (nitrogen-containing aromatic ring/metal element) is 0.1 or more and 30 or less, and more preferably 1 or more and 20 or less.

The temperature when subjecting the second catalyst raw material to the heat treatment is preferably 150°C or higher and 800°C or lower, more preferably 180°C or higher and 550°C or lower, and still more preferably 200°C or higher and 380°C or lower. Also, although there is no particular limitation on the time for which the second catalyst raw material is subjected to a heat treatment, it is, for example, 0.25 hours or more and 10 hours or less, preferably 0.5 hours or more and 8 hours or less, and more preferably 1 hour or more and 5 hours or less.

In addition, the above heat treatment may be carried out in an inert gas atmosphere such as argon or nitrogen gas, or may be carried out in a reducing atmosphere such as hydrogen.

The second catalyst raw material to be subjected to a heat treatment is preferably in powder or particle form. When the second catalyst raw material is in powder or particle form, the catalyst obtained by the heat treatment can also be in powder or particle form. Also, the second catalyst raw material to be subjected to a heat treatment more preferably consists of a metal precursor and porous carbon, or a metal precursor, porous carbon, and a nitrogen-containing compound.

The second catalyst raw material may be obtained by, for example, preparing a dilute solution of the second catalyst raw material and drying that dilute solution. In the dilute solution of the second catalyst raw material, each component (metal precursor and porous carbon, or metal precursor, nitrogen-containing compound, and porous carbon) is preferably dispersed or dissolved in a dilution solvent. By dispersing or dissolving each component in the dilution solvent, a second catalyst material in which each component has been homogeneously mixed can be obtained.

As the dilution solvent to be used to dilute the second catalyst raw material, water or an organic solvent can be used, and it is preferably water. Aqueous ammonia may also be used as the dilution solvent.

Specific examples of the organic solvent are as described above. As the dilution solvent, a mixed solvent of an organic solvent and water may also be used. Although there is no particular limitation on the concentration of the dilute solution of the second catalyst raw material, it is, for example, 0.01 to 25 g/L, and preferably 0.1 to 5 g/L.

### <<Metal salt>>

Examples of the metal salt to be used as the second catalyst include a metal nitrate, a metal sulfate, a metal chloride, a metal bromide, a metal iodide, and a metal acetate, among which a metal chloride and a metal nitrate are preferable, and a metal nitrate is more preferable. Specifically, examples of the metal nitrate include cobalt nitrate (Co(NO₃)₂), nickel nitrate (Ni(NO₃)₂), copper nitrate (Cu(NO₃)₂), rhodium nitrate (Rh(NO₃)₃), palladium nitrate (Pd(NO₃)₂), silver nitrate (AgNO₃), iridium nitrate (Ir(NO₃)₄), platinum nitrate (Pt(NO₃)₄), gold nitrate (AuNO₃), ruthenium nitrate (Ru(NO₃)₃), iron nitrate (Fe(NO₃)₃), manganese nitrate (Mn(NO₃)₂), zinc nitrate (Zn(NO₃)₂), chromium nitrate (Cr(NO₃)₃), and tin nitrate (Sn(NO₃)₄).

Also, specific examples of the metal chloride include PdCl₂, RuCl₃, IrCl₃, PtCl₄, and AuCl₃. Alternatively, the metal salt may be HAuCl₄ or the like. Among these, PdCl₂, HAuCl₄, and Ir(III)Cl₃ are preferable.

One type of second catalyst may be used alone, or two or more types thereof may be used in combination.

### (Electrode substrate)

In the present embodiment, the anode 17 may contain an electrode substrate (current collector). Note that the details of the electrode substrate (current collector) are as described for the cathode, and will thus be omitted. Note that, for the current collector of the anode 17, the same material as the current collector of the cathode may be used, or a different material may be used.

In the present embodiment, the second catalyst may be supported on the electrode substrate. Also, the second catalyst may be supported on the electrode substrate together with a catalyst additive and the like. Although there is no particular limitation on the method for supporting the second catalyst, or the second catalyst and catalyst additive (catalyst composition), on the electrode substrate, it is as described for the method for supporting the first catalyst on the electrode substrate, and the description therefor will thus be omitted.

### [Electrolytic solution]

In the present embodiment, the electrolytic solution contains a reactant and an electrolyte.

### (Electrolyte)

The electrolytic solution preferably contains a redox species as the electrolyte. The redox species may be dissolved in the reactant or in a mixture of the reactant and a solvent for electrolytic solution, which will described later. As the redox species, those with a molecular or ionic size smaller than the reactant, such as an alcohol-based compound described later, and with redox activity can be used. Specific examples of the redox species include a halide salt such as a metal halide salt, an organic redox, and a complex redox. Among these, from the viewpoint of increasing selectivity and productivity, a halide salt is preferable.

In the present embodiment, the electrolytic solution contains a redox species as the electrolyte, which produces a redox mediator (an oxidized product of halogen or the like) at the anode, and by that redox mediator, a carbonyl compound can be produced more efficiently from the carbon monoxide and the reactant in the presence of the second catalyst. Also, the redox mediator inhibits the reduction reaction of carbon dioxide when it comes into contact with the first catalyst, but in the present embodiment, the electrolytic solution has a convection flow, and the direction of the convection flow is controlled so that it comes into contact with the first catalyst and the second catalyst in this order, and therefore, the redox mediator described above is less likely to come into contact with the first catalyst and the reduction reaction of carbon dioxide is less likely to be inhibited.

Examples of the halide salt include a metal halide salt. Specific examples of the metal halide salt include a lithium halide salt such as lithium chloride, lithium bromide, and lithium iodide, a sodium halide salt such as sodium chloride, sodium bromide, and sodium iodide, a potassium halide salt such as potassium chloride, potassium bromide, and potassium iodide, a cesium halide salt such as cesium chloride, cesium bromide, and cesium iodide, and an ammonium halide such as ammonium chloride, ammonium bromide, and ammonium iodide.

Also, examples of the organic redox include a TEMPO-based radical compound such as 2,2,6,6-tetramethylpiperidine 1-oxyl (TEMPO) and 4-methoxy-2,2,6,6-tetramethylpiperidine 1-oxyl (MeO-TEMPO), and azaadamantane N-oxyl (AZADO).

In addition, examples of the complex redox include a palladium-based complex such as palladium acetylacetonate (Pd(OAc)₂) and tetrakis(triphenylphosphine)palladium (Pd(PPh₃)₄ complex), and a cobalt-based complex such as tris(2,2'-bipyridine)cobalt (Co(bpy)₃ complex) and tris[1,3-bis(4-pyridyl)propane]cobalt (Co(bpp)₃ complex).

Among the above, as the redox species, from the viewpoint of increasing the selectivity for a carbonyl compound, a metal halide salt is preferable, among which a metal chloride salt and a metal bromide salt are more preferable, and a metal bromide salt is still more preferable.

Accordingly, in the electrolytic solution, halogen ions such as chlorine ions or bromine ions are contained as the redox ions, and particularly preferably, bromine ions are contained.

Preferable specific examples of the metal chloride salt include lithium chloride, potassium chloride, and sodium chloride, among which sodium chloride is preferable from the viewpoint of ease of availability. Preferable specific examples of the metal bromide salt include lithium bromide, potassium bromide, and sodium bromide, among which sodium bromide is particularly preferable from the viewpoint of increasing the selectivity for a carbonyl compound. One type of redox species may be used alone, or two or more types thereof may be used in combination.

There is no particular limitation on the concentration of the redox species in the electrolytic solution, and it may be adjusted to the extent that the synthesis of an oxygen-containing organic material, such as a carbonyl compound, proceeds appropriately. The specific concentration of the redox species is, for example, 0.001 M or more and 5.0 M or less, preferably 0.01 M or more and 1.0 M or less, and more preferably 0.05 M or more and 0.5 M or less.

### (Reactant)

The reactant is a compound that serves as a raw material for an oxygen-containing organic material such as a carbonyl compound. The reactant is changed depending on the type of target product to be produced. For example, in the case where an organic carbonate, an organic oxalate, or both of them are to be produced, an alcohol-based compound may be used as the reactant. Also, in the case where a urea-based compound is to be produced, an amine-based compound may be used as the reactant. In addition, in the case where a carbonyl halide such as phosgene is to be produced, a halogen or a metal halide salt may be used as the reactant, as will be described later. Among the above, it is preferable to use an alcohol-based compound as the reactant.

### (Alcohol-based compound)

The alcohol-based compound is a reactant that reacts with the carbon monoxide in the electrochemical reaction apparatus to produce an organic carbonate, an organic oxalate, or both of them.

The alcohol-based compound may be any of solid, liquid, and gas in the environment where the electrochemical reaction is carried out in the electrochemical reaction apparatus, but it is preferably liquid. The electrochemical reaction apparatus can be easily filled with the alcohol-based compound that is liquid even without the use of a solvent for electrolytic solution, which will be described later.

The alcohol-based compound is a compound that has at least one hydroxy group, and more specifically, is a compound represented by the following general formula (1). Note that, in the present specification, the "alcohol-based compound" is a concept that also includes aromatic hydroxy compounds in which a hydroxy group is directly bonded to an aromatic ring such as a benzene ring as represented by phenol, as will be described later.

ROH (1)

wherein R represents an organic group having 1 to 15 carbon atoms.

Examples of the organic group having 1 to 15 carbon atoms represented by R in the above general formula (1) include a hydrocarbon group having 1 to 15 carbon atoms. Examples of the hydrocarbon group include an alkyl group having 1 to 15 carbon atoms or an alkenyl group having 2 to 15 carbon atoms, as well as an aryl group having 6 to 15 carbon atoms.

Examples of the alkyl group having 1 to 15 carbon atoms include a methyl group, an ethyl group, various propyl groups, various butyl groups, various pentyl groups, various hexyl groups, various heptyl groups, various octyl groups, various nonyl groups, various decyl groups, various dodecyl groups, and various pentadecyl groups.

Examples of the alkenyl group having 2 to 15 carbon atoms include a vinyl group, various propynyl groups, various butynyl groups, various pentynyl groups, various hexenyl groups, various heptenyl groups, various octenyl groups, various nonenyl groups, various decenyl groups, various dodecenyl groups, and various pentadecenyl groups.

Note that the term "various" means various isomers that include n-, sec-, tert-, and iso-isomers. Also, the alkyl group or alkenyl group may be any of linear, branched, and cyclic forms.

Examples of the aryl group having 6 to 15 carbon atoms include a phenyl group and a naphthyl group. Note that the above hydrocarbon group may have a substituent, in which case the number of carbon atoms including the substituent is 1 to 15.

Also, the organic group having 1 to 15 carbon atoms in the general formula (1) may contain a heteroatom such as a nitrogen atom, an oxygen atom, a sulfur atom, a phosphorus atom, or a halogen atom.

Among these, an oxygen atom is preferable. In the case where the organic group has an oxygen atom, that oxygen atom is preferably an oxygen atom either in a hydroxy group or in an ether bond. Accordingly, R is preferably a hydrocarbon group that has at least any of a hydroxy group and an ether bond. Also, it is preferable that there is one hydroxy group in R. In other words, the alcohol-based compound may have two hydroxy groups.

As the alcohol-based compound having two hydroxy groups, more specifically, a group represented by the following formula (1-1) is preferable.

HO-R¹¹-OH (1-1)

Note that, in the formula (1-1), R¹¹ is a divalent saturated hydrocarbon group having 2 to 15 carbon atoms, while the number of carbon atoms in R¹¹ is preferably 2 to 4, and more preferably 2 to 3.

Among the above, as the compound represented by the above general formula (1), those are preferable in which R is an alkyl group having 1 to 8 carbon atoms, an alkenyl group having 2 to 8 carbon atoms, or an aryl group having 6 to 8 carbon atoms, and those represented by the general formula (1-1) are also preferable in which R¹¹ has 2 to 4 carbon atoms.

Among these, compounds in which R is an alkyl or an aryl group are more preferable, and in particular, compounds in which R is an alkyl group are still more preferable. Also, the alkyl group more preferably has 1 to 3 carbon atoms, still more preferably has 1 or 2 carbon atoms, and most preferably has 1 carbon atom.

Specifically, from the viewpoint of reactivity and production efficiency, and the like, methanol, ethanol, phenol, 1-propanol, ethylene glycol, propylene glycol, and the like are preferable among which methanol is more preferable.

Note that one type of alcohol-based compound may be used alone, or two or more types thereof may be used in combination.

In the case where an alcohol-based compound is used as the reactant, in the electrochemical reaction apparatus 10, a reaction is generally carried out in which an organic carbonate is produced from the carbon monoxide and the alcohol-based compound (also referred to as first reaction). However, a reaction may also be carried out in which an organic oxalate is produced from the carbon monoxide and the alcohol-based compound (also referred to as second reaction), and both of the first and second reactions may be carried out, but it is preferable that at least the first reaction is carried out.

The above first reaction is a carbonylation reaction in which an organic carbonate is produced, and specifically, an organic carbonate ((RO)₂CO) is produced by the reaction represented by the following formula (i).

CO + 2ROH → (RO)₂CO + 2H⁺ + 2e⁻ (i)

Note that R in (i) is the same as described above, but preferably R is an alkyl group having 1 to 8 carbon atoms, an alkenyl group having 2 to 8 carbon atoms, or an aryl group having 6 to 8 carbon atoms, more preferably an alkyl group or an aryl group, and still more preferably an alkyl group. The alkyl group more preferably has 1 to 3 carbon atoms, still more preferably has 1 or 2 carbon atoms, and most preferably has 1 carbon atom.

However, as described above, two or more types of alcohol compounds may be used in combination, in which case two R in one molecule of (RO)₂CO may be different from each other.

Also, in the case where ROH is represented by the general formula (1-1), an organic carbonate is produced by the reaction represented by the following formula (ii).

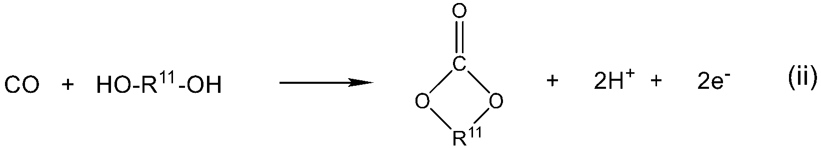

Note that R¹¹ in the formula (ii) is the same as described above, but the number of carbon atoms in R¹¹ is preferably 2 to 4, more preferably 2 to 3, and still more preferably 2.

Specific preferable examples of the organic carbonate include one or two or more selected from the group consisting of dimethyl carbonate, diethyl carbonate, ethylene carbonate, dipropyl carbonate, propylene carbonate, diphenyl carbonate, ethyl methyl carbonate, methyl propyl carbonate, and ethyl propyl carbonate, among which dimethyl carbonate is more preferable.

The second reaction is a reaction in which an organic oxalate represented by the following formula (2) is produced from the carbon monoxide and the alcohol-based compound. Specifically, the organic oxalate represented by the formula (2) may be synthesized by the reaction represented by the following formula (iii). 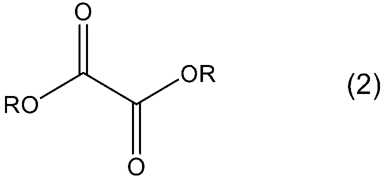 wherein, in the formula (2), R is the same as described above. However, two R in one molecule may be identical to or different from each other. 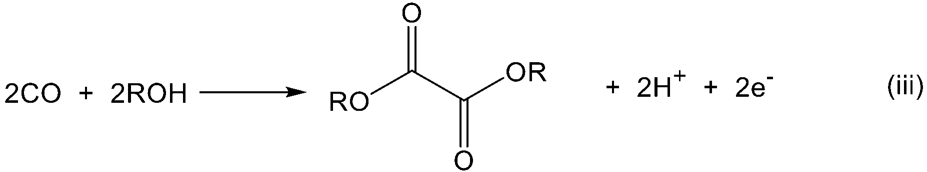 wherein, in the formula (iii), R is the same as described above.

Also, in the case where ROH is represented by the general formula (1-1), an organic oxalate represented by the following formula (2-1) is produced by the reaction represented by the following formula (iv). 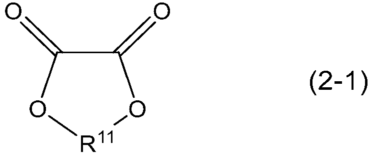 wherein, in the formula (2-1), R¹¹ is the same as described above. 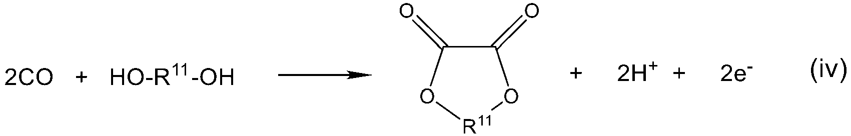 wherein, in the formula (iv), R¹¹ is the same as described above.

Specific preferable examples of the organic oxalate include one or two or more selected from the group consisting of dimethyl oxalate, diethyl oxalate, ethylene oxalate, dipropyl oxalate, propylene oxalate, diphenyl oxalate, ethyl methyl oxalate, methyl propyl oxalate, and ethyl propyl oxalate. Among these, dimethyl oxalate is more preferable.

### (Amine-based compound)

The amine compound to be used as the reactant is an organic compound that has at least one amino group, and is preferably a compound represented by the following formula (3).

R²NH₂ (3)

wherein R² represents an organic group having 1 to 15 carbon atoms.

Examples of the organic group having 1 to 15 carbon atoms represented by R² in the above formula (3) include a hydrocarbon group having 1 to 15 carbon atoms, the specific description for which is the same as that for the hydrocarbon group in R¹ described above.

Also, the organic group having 1 to 15 carbon atoms in the formula (3) may contain a heteroatom such as a nitrogen atom, an oxygen atom, a sulfur atom, and a phosphorus atom. Among these, a nitrogen atom is preferable, and the nitrogen atom is preferably a nitrogen atom in an amino group. Accordingly, R² is preferably a hydrocarbon group that has an amino group. More specifically, an aminoalkyl group having 1 to 15 carbon atoms is preferable.

By using an amine-based compound as the reactant, a urea-based compound, which has a urea bond (-NH-CO-NH-), can be synthesized.

### (Halogen)

In the case where the target product is a carbonyl halide, a halogen may be used as the reactant. Examples of the halogen include chlorine and bromine. Note that phosgene is obtained when the halogen is chlorine, and carbonyl bromide is obtained when the halogen is bromine. Instead of the halogen, halogen may be produced by the electrochemical reaction in the electrochemical reaction apparatus 10 from the metal halide salt used as the electrolyte. Accordingly, in the case where a carbonyl halide is to be produced, a metal halide salt may be used both as the reactant and as the electrolyte (redox species).

### [Solvent for electrolytic solution]

In the case where the reactant described above is solid or gas, in the case where it is necessary to enhance the solubility of the redox species, or in other cases, the electrolytic solution may further contain a solvent (also referred to as "solvent for electrolytic solution"). In such cases, the reactant may be provided in the electrochemical reaction apparatus as a mixture with the solvent. Of course, even in the case where the reactant is liquid, it may be provided in as a mixture with the solvent.

The solvent for electrolytic solution can be selected as appropriate from the group consisting of solvents normally used in electrochemical reactions, and examples thereof include a nitrile-based solvent such as acetonitrile, a carbonate-based solvent such as ethylene carbonate, propylene carbonate, butylene carbonate, vinylene carbonate, dimethyl carbonate, ethyl methyl carbonate, and diethyl carbonate, a lactone-based solvent such as γ-butyrolactone, an ether-based solvent such as 1,2-dimethoxyethane, 1-ethoxy-2-methoxyethane, 1,2-diethoxyethane, tetrahydrofuran, and 2-methyltetrahydrofuran, a phosphate ester solvent, a phosphoric acid type, a sulfolane-based solvent, and a pyrrolidone. One of these solvents may be used alone, or two or more thereof may be used in combination.

### <Second embodiment>

Next, the second embodiment of the present invention will be described. Fig. 2 shows an electrochemical reaction apparatus (electrochemical cell) 20 according to the second embodiment of the present invention. In the first embodiment, the first and second catalysts are arranged on the identical plane and the convection flow F flows in parallel to that plane, but in the present embodiment, first and second catalysts 26 and 27 are arranged so that they face each other and a convection flow F is formed in such a direction that an electrolytic solution 23 comes into contact with the first and second catalysts 26 and 27 in this order. In the following, the points of difference in the second embodiment from the first embodiment will be described, and the parts where description is omitted are the same as in the first embodiment.

In the second embodiment, an anode 22 and a cathode 21 are provided on wall surfaces 20A and 20B that face each other in the Y direction of a reaction chamber 20, respectively. Between the anode 22 and the cathode 21, a voltage is applied by a power source. The anode 22 and the cathode 21 have a second catalyst 27 and a first catalyst 26, respectively, in the same manner as in the first embodiment. Accordingly, the cathode 21 and the anode 22, that is, the first catalyst 26 and the second catalyst 27, face each other in the Y direction.

Furthermore, on each of the wall surfaces 20A and 20B, which face each other in the Y direction, a discharge port 25 and a supply port 24 are provided, respectively. As a result, the supply port 24 is arranged on the cathode 21 (first catalyst) side and the discharge port 25 is arranged on the anode 22 (second catalyst) side and they are displaced from each other in the Y direction. Then, since the electrolytic solution 23 that has been supplied from the supply port 24 is discharged from the discharge port 25, the convection flow F of the electrolytic solution 23 is formed in a direction along the Y direction (that is, from the cathode 21 toward the anode 22), and the electrolytic solution 23 comes into contact with the first catalyst 26 and the second catalyst 27 in this order. Therefore, the reduction of carbon dioxide is catalyzed by the first catalyst 26, and a reduced product of carbon dioxide (carbon monoxide) is produced at the cathode 21. Meanwhile, at the anode 22, an oxygen-containing organic material, such as a carbonyl compound, is produced from the reduced product (carbon monoxide) produced at the cathode 21 and the reactant by the second catalyst 27.

As described above, in the present embodiment as well, the electrochemical reaction apparatus 20 can efficiently synthesize an oxygen-containing organic material, such as a carbonyl compound, from carbon dioxide, despite its simple configuration. Then, by circulating the electrolytic solution 23, the production rate of an oxygen-containing organic material can be enhanced.

Note that the supply port 24 and the discharge port 25 are positioned outside of the cathode 21 and the anode 22, and are arranged so as to interpose them therebetween when viewed in the Y direction. Such an arrangement makes it so much easier for the electrolytic solution 23 that is allowed to flow by the convection flow F to come into contact with the first catalyst 26 possessed by the cathode 21 and the second catalyst 27 possessed by the anode 22, which makes it so much easier to enhance the production rate of an oxygen-containing organic material. However, the positions of the supply port 24 and the discharge port 25 are not limited to the configuration shown in Fig. 2, and there is no particular limitation on them as long as the direction of the convection flow F is formed so that the electrolytic solution 23 comes into contact with the first catalyst 26 and the second catalyst 27 in this order.

### <Third embodiment>

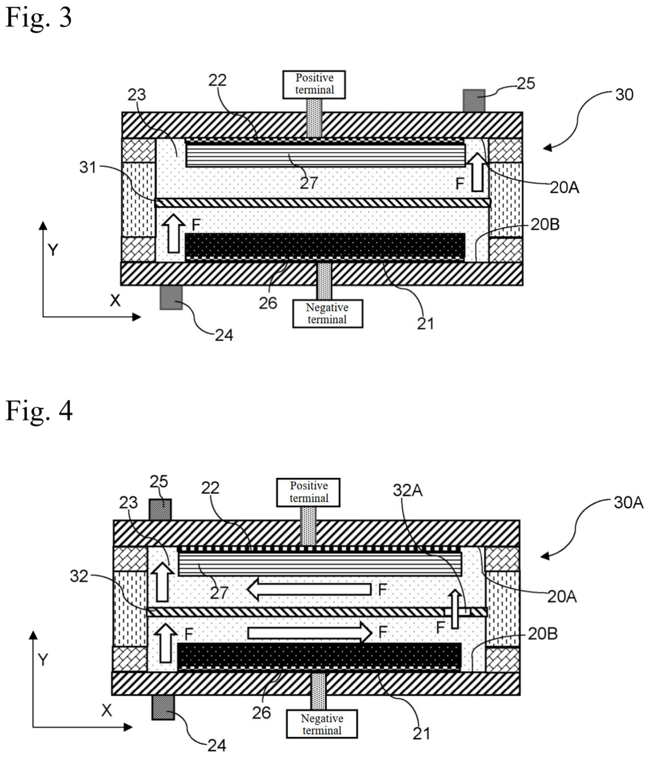

Fig. 3 shows an electrochemical reaction apparatus (electrochemical cell) 30 according to the third embodiment of the present invention. In the following, the points of difference in the third embodiment from the second embodiment will be described, and the parts where description is omitted are the same as in the second embodiment. The third embodiment differs from the second embodiment in that an electrochemical reaction apparatus 30 comprises a rectifying layer 31. Note that, in the third embodiment, identical components to those in the second embodiment are marked with identical symbols.

The rectifying layer 31 is arranged between the cathode 21 (that is, the first catalyst 26) and the anode 22 (that is, the second catalyst 27), and is provided so as to separate the cathode 21 side region from the anode 22 side region.

The rectifying layer 31 is a layer that regulates the direction of the convection flow F of the electrolytic solution 23. Examples of the rectifying layer 31 include an insulating mesh filter, and specific examples thereof include a resin mesh filter. Examples of the resin mesh filter is a filter of which a resin sheet on which holes have been made as appropriate by punching or other methods. There is no particular limitation on the shape of the holes, and they may be in the shape of circles or ovals, polygons such as triangles, squares or pentagons, or any other shapes. There is no particular limitation on the mesh (openings), but the diameter of the holes is preferably 0.1 µm or more and 5000 µm or less, and more preferably 1 µm or more and 3000 µm or less. Note that the diameter of the holes means the longest diameter (long diameter) in the holes in the case where they are other than circles. The same applies below.

Examples of the resin sheet include a fluoropolymer sheet such as polytetrafluoroethylene (PTFE) and perfluoroalkoxy alkane (PFA).

In the present embodiment, the rectifying layer 31 controls the flow of the convection flow F so that it follows the Y direction, that is, from the cathode 21 toward the anode 22. Accordingly, in the present embodiment, provision of the rectifying layer 31 makes it easier for the convection flow F to be formed along the Y direction, and thus makes it easier for the electrolytic solution 23 to flow to come into contact with the first catalyst 26 and the second catalyst 27 in this order, enabling even more efficient production of an oxygen-containing organic material.

Note that the rectifying layer is not limited to a mesh filter, and for example, it may be a film in which holes other than a mesh shape have been made in a partition wall thereof, or for example, it may be one in which one or several holes have been made only in a part of the region of the partition wall. More specifically, examples of the rectifying layer include one in which one or several holes have been made in a part of the region of the resin sheet. In this case, the size of the holes is, for example, a diameter of 0.1 µm or more and 5000 µm or less, and preferably a diameter of 1 µm or more and 3000 µm or less. An electrochemical reaction apparatus (electrochemical cell) 30A that uses such a rectifying layer 32 is shown in Fig. 4.

In the example shown in Fig. 4, the rectifying layer 32 is provided with hole(s) 32A in a part of the region, and the hole(s) 32A are at a position apart from the supply port 24 and the discharge port 25. Specifically, when viewed in the Y direction, the hole(s) 32A and the supply port 24 may be arranged so as to interpose a part (preferably 50% or more of the portion along the X direction) or all of the portion along the X direction of the cathode 21 therebetween. Similarly, the hole(s) 32A and the discharge port 25 may be arranged so as to interpose a part (preferably 50% or more of the portion along the X direction) or all of the portion along the X direction of the anode 21 therebetween. Such an arrangement of the hole 32A, the supply port 24, and the discharge port 25 causes the convection flow F of the electrolytic solution 23 to be formed so that it comes into contact with a large portion of the first catalyst 26 of the cathode 21 and the second catalyst 27 of the anode 22. Therefore, even the example shown in Fig. 4 can also efficiently carry out the reduction of carbon dioxide and the production of an oxygen-containing organic material.

### <Fourth embodiment>

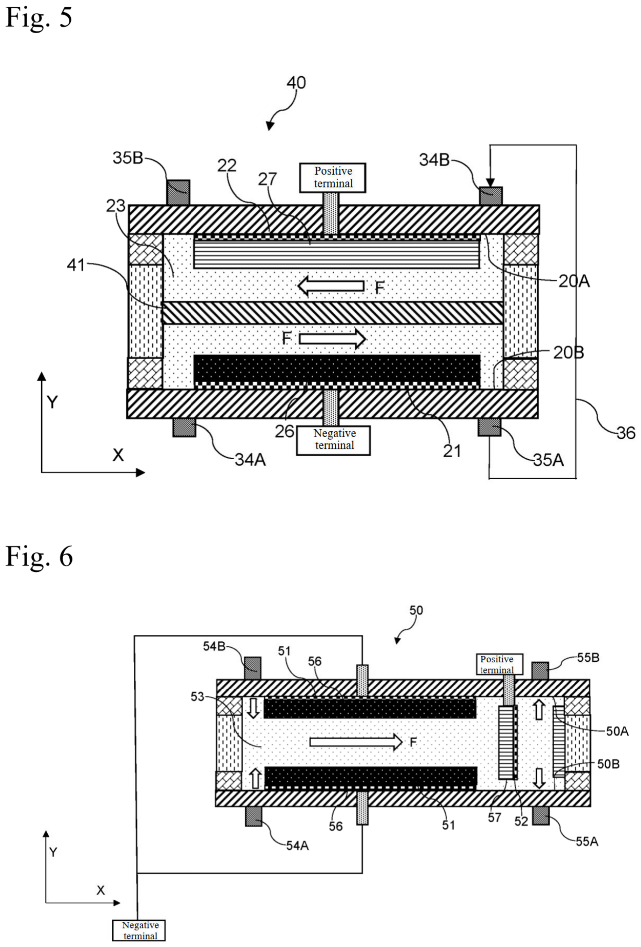

Fig. 5 shows an electrochemical reaction apparatus (electrochemical cell) 40 according to the fourth embodiment of the present invention. In the following, the points of difference in the fourth embodiment from the second embodiment will be described, and the parts where description is omitted are the same as in the second embodiment. Note that, in the fourth embodiment, identical components to those in the second embodiment are marked with identical symbols.

The fourth embodiment differs from the second embodiment in that the electrochemical reaction apparatus 40 comprises an isolating layer 41, and is also provided with first and second supply ports 34A and 34B as supply ports and first and second discharge ports 35A and 35B as discharge ports.

The isolating layer 41 is arranged between the cathode 21 (that is, the first catalyst 26) and the anode 22 (that is, the second catalyst 27), and is provided to isolate the cathode 21 side region from the anode 22 side region.

The isolating layer 41 may be one that isolates the carbon monoxide generated by the first catalyst 26 (cathode 21) from an oxidized product generated by the second catalyst 27 (anode 22). Also, the isolating layer 41 may be one that allows ions to permeate but does not allow the electrolytic solution 23, carbon monoxide, and oxidized product to permeate, and specifically, an ion exchange membrane is preferable. As the ion exchange membrane, a solid membrane is used. Examples thereof include a cation exchange membrane that allows cations such as protons to permeate, and an anion exchange membrane that allows anions such as hydroxide ions to permeate, but from the viewpoints of ion conductivity and cost, a cation exchange membrane is preferable.

Examples of the cation exchange membrane include those having at least any of sulfonyl groups, carboxyl groups, phosphate groups, and silicate groups as functional groups. Examples of the cation exchange membrane having sulfonyl groups as functional groups include a hydrocarbon resin-based polysulfonic acid, such as polyethylene sulfonic acid and fullerene-bridged polysulfonic acid, and a fluoropolymer-based sulfonic acid, such as perfluoroethylene sulfonic acid. Examples of the perfluoroethylene sulfonic acid include a copolymer of tetrafluoroethylene and perfluoro[2-(fluorosulfonylethoxy)propyl vinyl ether], and examples of commercially available products include "Nafion" (TM of DuPont de Nemours, Inc.).

Also, examples of those having carboxyl groups as functional groups include a polycarboxylic acid, such as polyacrylic acid. As those having phosphate groups or silicate groups as functional groups, heteropoly acids such as silicotungstic acid and phosphotungstic acid can also be used. Furthermore, as the cation exchange membrane, phosphate glasses such as SiO₂-P₂O₅ and ceramics such as perovskite type oxides can also be used.

Also, examples of the anion exchange membrane include resins having a quaternary ammonium salt such as poly(styrylmethyltrimethylammonium chloride) polyethers, and polymers having imidazolium groups. Examples of the resins having an ammonium salt include "FAA-3-50" from FuMA-Tech GmbH and "TM1 Durion Grade" from Orion Polymer. Examples of the polymers having imidazolium groups include styrene-based polymers having imidazolium groups. Specific examples thereof include a copolymer of styrene and 1-(p-vinylbenzyl)-3-methylimidazolium (PSMIM), a copolymer of styrene and 1-(p-vinylbenzyl)-tetramethyl-imidazolium (PSTMIM), and a copolymer of styrene and 1-(p-vinylbenzyl)-2,3-dimethyl-imidazolium (PSDMIM).

A first supply port 34A and a first discharge port 35A are provided on the wall surface 20B, arranged outside of the cathode 21 on the wall surface 20B, and arranged so as to interpose the cathode 21 therebetween. Also, a second supply port 34B and a second discharge port 35B are arranged outside 21 of the anode 22 on the wall surface 20A, and are arranged so as to interpose the anode 22 therebetween. Furthermore, the first discharge port 35A is connected to the second supply port 34B via a connecting path 36.

By having the above configuration, the electrolytic solution 23 is supplied from the first supply port 34A, and in the cathode 21 side region, allowed to flow along the surface of the cathode 21 to come into contact with the first catalyst 26 of the cathode 21, and then supplied to the anode 22 side region via the connecting path 36. Then, in the anode 22 side region, the electrolytic solution 23 is allowed to flow along the surface of the anode 22 to come into contact with the second catalyst 27 of the anode 22, and then discharged from the second discharge port 35B. Accordingly, in the present embodiment as well, the electrolytic solution 23 has a direction of the convection flow F that comes into contact with the first catalyst 26 and the second catalyst 27 in this order. Also, in the present embodiment as well, the electrolytic solution 23 that has been discharged from the second discharge port 35B may be circulated and resupplied from the first supply port 34A, and the circulation may be repeated.

As described above, the isolating layer 41 does not allow the electrolytic solution 23 and other materials to permeate, but it does allow ions to permeate, and therefore, in the present embodiment as well, when a voltage is applied between the cathode 21 and the anode 22, electrochemical reactions occur at the cathode 21 and the anode 22. Therefore, in the present embodiment, carbon dioxide is reduced to produce a reduced product (carbon monoxide) in the cathode 21 side region, while an oxygen-containing organic material, such as a carbonyl compound, is produced from the reduced product and the reactant contained in the electrolytic solution 23 in the anode 22 side region, and is discharged from the second discharge port 35B.

Here, the isolating layer 41 does not allow the carbon monoxide produced in the cathode 21 side region to permeate to the anode 22 side, and does not allow the oxidized product (redox mediator or the like) generated on the anode 22 side to permeate to the cathode 21 side either. Therefore, on the cathode 21 side, inhibition of the reaction by the oxidized product and occurrence of a byproduct due to the oxidized product are prevented. Similarly, on the anode 22 side as well, inhibition of the production reaction for an oxygen-containing organic material by carbon monoxide and occurrence of a byproduct are prevented either, enabling more efficient production of an oxygen-containing organic material.

Accordingly, in the present embodiment, the electrochemical reaction apparatus 40 can synthesize an oxygen-containing organic material more efficiently using carbon dioxide as a raw material. Also, by circulating the electrolytic solution 23, the production rate of an oxygen-containing organic material can be improved.

### <Fifth embodiment>

Next, an electrochemical reaction apparatus according to the fifth embodiment of the present invention will be described. Fig. 6 shows an electrochemical reaction apparatus (electrochemical cell) 50 according to the fifth embodiment. In the following, the points of difference in the fifth embodiment from the first embodiment will be described. The electrochemical reaction apparatus 50 according to the fifth embodiment of the present invention comprises cathodes 51, an anode 52, and an electrolytic solution 53 that contains a reactant and an electrolyte. A plurality of cathodes 51 (in the present embodiment, two) are provided, each of which has a first catalyst 56 that reduces carbon dioxide to carbon monoxide. The anode 52 contains a second catalyst 57. In the present embodiment, there is one anode 52.

The cathodes 51 and 51 are provided on wall surfaces 50A and 50B that face each other in the Y direction, respectively, and are thus arranged in parallel to each other. The cathodes 51 are parallel to the X direction. With such an aspect, the plurality of cathodes 51 can be said to be in parallel. On the other hand, the anode 52 is arranged in parallel to the Y direction, and the anode 52 is arranged orthogonally to each of the cathodes 51. Note that the anode 52 is attached to the wall surface 50A of the electrochemical reaction apparatus 50, but it is not limited to such an aspect.

The first catalyst 56 and the second catalyst 57 are supported in the form of a layer, for example, on the surface of the cathodes 51 and the anode 52, respectively, and accordingly, the first catalyst 56 of the cathodes 51 and the second catalyst 57 of the anode 52 are arranged orthogonally to each other in the same manner as the cathodes 51 and the anode 52.

The wall surface 50B is provided with a first supply path 54A and a first discharge port 55A, and the wall surface 50A is provided with a second supply path 54B and a second discharge port 55B. The supply paths 54A and 55A and the discharge ports 55A and 55B are arranged outside of the cathodes 51 (first catalyst 56) and the anode 52 (second catalyst 57), and are arranged so as to interpose them therebetween when viewed in the Y direction.

In the present embodiment, the electrolytic solution 53 is supplied from the first supply path 54A and the second supply path 54B, which are arranged on the cathode 51 side, and discharged from the second discharge path 55A and the second discharge path 55B, which are arranged on the anode 52 side. Accordingly, in the present embodiment, a convection flow F is formed so that the electrolytic solution 23 is allowed to flow along the cathodes 51 and the anode 52, which are aligned along the X direction, from the cathodes 51 toward the anode 52. As a result, in the present embodiment as well, the direction of the convection flow F is formed so that the electrolytic solution 53 comes into contact with the cathodes 51 (first catalyst 56) and the anode 52 (second catalyst 57) in this order, and as in each of the embodiments described above, the electrochemical reaction apparatus 50 can efficiently produce an oxygen-containing organic material, such as a carbonyl compound. Also, in the present embodiment as well, by circulating the electrolytic solution 53, the production rate of an oxygen-containing organic material, such as a carbonyl compound, can be improved.

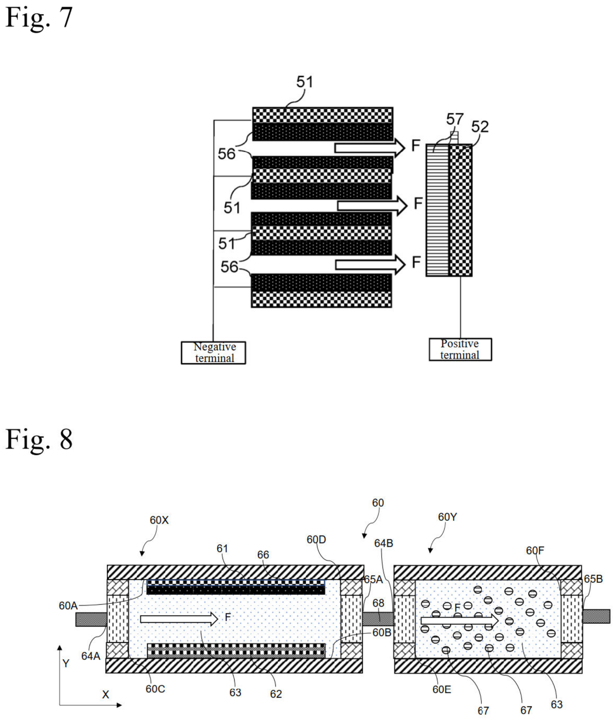

Note that, in the present embodiment, there is shown an aspect in which two cathodes 51 are provided, but there may be one cathode 51, or three or more cathodes may be provided, as shown in Fig. 7, for example. In the case where three or more are provided, the cathodes 51 on both sides of a plurality of cathodes 51 aligned in parallel may be attached to the wall surfaces of the reaction chamber, for example, as shown in Fig. 6. The cathodes 51 other than those on both sides may be supported by holders or the like provided inside the reaction chamber (not shown in the figure).

Furthermore, Fig. 7 shows an aspect in which the cathodes 51 on both sides have a catalyst layer by the first catalyst 26 formed on only one surface, and the cathodes 51 other than those on both sides have catalyst layers formed on both surfaces, but there is no limitation to the present aspect.

Moreover, in the present embodiment, the cathodes 51 and the anode 52 do not need to be orthogonal, and the cathodes 51 may be arranged inclined with respect to the anode 52. As a result, the first catalyst 56 of the cathodes 51 is arranged so that it is inclined with respect to the second catalyst 57 of the anode 52. When arranged inclined, the inclination angle may be 1° or more and 179° or less, but it is preferably 60° or more and 120° or less, and still more preferably 80° or more and 100° or less.

Also, in the present embodiment, a plurality of anodes 52 may be provided as well. Furthermore, although two supply ports and two discharge ports were each provided, the number of supply ports and discharge ports is not limited, and it may be one, for example.

### <Sixth embodiment>

Next, the sixth embodiment of the present invention will be described. Fig. 8 shows an electrochemical reaction apparatus 60 according to the sixth embodiment. In the following, the points of difference in the sixth embodiment from the first embodiment will be described. The electrochemical reaction apparatus 60 according to the sixth embodiment comprises a cathode 61, an anode 62, and an electrolytic solution 63 that contains a reactant and an electrolyte. The cathode 61 has a first catalyst 66 in the same manner as in the first embodiment.

On the other hand, in the present embodiment, the anode 62 may not contain a second catalyst. In order to make the anode contain a catalyst, as described above, a process such as supporting the catalyst on the electrode is necessary. However, if the anode does not contain a catalyst, such a process becomes unnecessary and the electrochemical reaction apparatus (electrochemical cell) can be made to have a simplified configuration.

The electrochemical reaction apparatus 60 according to the present embodiment comprises a second reaction section in addition to the electrochemical cell (first reaction section), and a series of reactions to obtain an oxygen-containing organic material from carbon dioxide are carried out in the first reaction section and the second reaction section.

The electrochemical reaction apparatus 60 according to the present embodiment comprises a first reaction chamber (first reaction section) 60X that comprises the cathode 61 and the anode 62, and a second reaction chamber (second reaction section) 60Y. The first reaction chamber 60X is an electrochemical cell and has wall surfaces 60A and 60B that face each other in the Y direction, with the cathode 61 and the anode 62 attached to the wall surfaces 60A and 60B, respectively, and the cathode 61 and the anode 62 are provided so that they face each other in the Y direction. Between the cathode 61 and the anode 62, a voltage is applied by a power source not shown in the figure. Also, on wall surfaces 60C and 60D that face each other in the X direction of the first reaction chamber 20X, a first supply port 64A and a first discharge port 65A are provided, respectively. As a result, the first supply port 64A and the first discharge port 65A are arranged outside of the anode 62 and cathode 61, so that they interpose the anode 62 and the cathode 61 therebetween when viewed along the Y direction.

In the present embodiment, the first reaction chamber 60X may be a one-compartment type electrochemical cell (electrolytic cell). That is, the first reaction chamber 10X is constituted by one electrolytic compartment without being divided into an anode compartment and a cathode compartment by an isolating layer such as an ion exchange membrane, and the electrolytic compartment is provided with both the cathode 11 and the anode 12, and is also filled with the electrolytic solution 13.

The second reaction chamber 60Y is filled with the electrolytic solution 63 and the electrolytic solution 63 contains a second catalyst 67. The second catalyst 67 may be dispersed or dissolved in the electrolytic solution 63. Also, the second reaction chamber 60Y is provided with a second supply port 64B and a second discharge port 65B on wall surfaces 60E and 60F that face each other, respectively. The second supply port 64B is connected to the first discharge port 65A via a connecting path 68. Note that there is no particular limitation on the connecting path, as well as on the circulating path, supply path, branched path, and discharge path described later, but they may be constituted by piping or the like.

It is preferable that a filter not shown in the figure is attached to the second discharge port 65B. The attachment of a filter to the second discharge port 65B prevents the second catalyst 67 in the electrolytic solution 63, which is filled in the second reaction chamber 60Y, from being discharged from the second discharge port 65B together with the electrolytic solution 63. Also, in the present embodiment, the second supply port 64B may be provided with a filter as well. Provision of a filter at the second supply port 64B can prevent the second catalyst 67 in the second reaction chamber 60Y from flowing backward and mixing into the first reaction chamber 60X.

The filter may be anything as long as it does not allow the second catalyst 67 to permeate, and for example, in the case where the second catalyst is dispersed in the electrolytic solution 23, it may be a membrane filter, a mesh filter, or the like. On the other hand, in the case where the second catalyst 67 is dissolved in the electrolytic solution 63, a chemisorption filter, a physical adsorption filter, or the like may be used.

Also, the inside of the second reaction chamber 60Y may be provided with a stirring device, such as a stirring blade or a stirrer, to stir the electrolytic solution 63 inside. Stirring the inside of the second reaction chamber 60Y makes it easier to diffuse the second catalyst 67 in the electrolytic solution 63 even in the case where the second catalyst 67 is not dissolved in the electrolytic solution 63, which makes it easier to enhance the reaction efficiency in the second reaction chamber 60Y. However, the second reaction chamber 60Y may not be provided with a stirring device. Even without provision of a stirring device, a convection flow F of the electrolytic solution 63 can diffuse the second catalyst 67 in the electrolytic solution 63. In addition, in the case where the second catalyst 67 is dissolved in the electrolytic solution 63, the second catalyst 67 can be diffused in the electrolytic solution 63 even without stirring.

With the above configuration, in the present embodiment, there are provided the first reaction section (first reaction chamber 60X, electrochemical cell), which comprises the cathode 61 comprising the first catalyst and the anode 62, and the second reaction section (second reaction chamber 60Y), which comprises the second catalyst 67.

The electrolytic solution 63 is allowed to flow from the first supply port 64A of the first reaction chamber 60X toward the first discharge port 65A, while the electrolytic solution 63 that has discharged from the first discharge port 65A is supplied to the second reaction chamber 60Y via the second supply port 64B. Then, in the second reaction chamber 60Y, the electrolytic solution 63 is discharged from the second discharge port 65B. Also, the electrolytic solution 63 that has been discharged from the second discharge port 65B may be circulated and resupplied from the first supply port 64A via a circulating path not shown in the figure, and the circulation may be repeated.

Note that, by allowing the electrolytic solution 63 to flow from the supply port 64A to the discharge port 65A in the first reaction chamber 60X, the convection flow F is formed inside the electrochemical cell so as to be oriented in the X direction. In other words, the convection flow F is formed so that the electrolytic solution 63 flows in a direction that crosses the direction in which the electrodes face each other.

In the present embodiment, by allowing the electrolytic solution 63 to flow as described above, the electrolytic solution 63 has a direction of the convection flow F that flows in the order of the first reaction section (first reaction chamber 60X, electrochemical cell) and the second reaction section (second reaction chamber 60Y). Then, at the cathode 61, the reduction of carbon dioxide is catalyzed by the first catalyst 66 to produce a reduced product of carbon dioxide (carbon monoxide). The electrochemical reaction that is carried out at the cathode is typically as in the above formula (A).

On the other hand, an oxidized product (for example, a redox mediator), which is an active intermediate species, is produced at the anode 62. In other words, at the anode 62, the redox species constituted by the electrolyte is converted from a reduced species to an oxidized product (for example, a redox mediator), which is an active intermediate species. In the case where the electrolyte is a brominated salt, the reaction that is carried out at the anode 62 is as in the following formula (B) and bromine is produced as the oxidized product.

2Br⁻ → Br₂ + 2e⁻ (B)

The carbon monoxide and active intermediate species (oxidized product) produced in the first reaction chamber 60X are supplied to the second reaction section (second reaction chamber 60Y) by the convection flow F together with the electrolytic solution 63. Then, in the second reaction section (second reaction chamber 60Y), an oxygen-containing organic material, such as a carbonyl compound, is produced from the carbon monoxide, active intermediate species, and reactant by the action of the second catalyst 67.

Note that the electrolytic solution 63 to be supplied to the second reaction chamber 60Y normally contains carbon dioxide in addition to the carbon monoxide. Also, the carbon monoxide may be dissolved in the electrolytic solution 63 or may be present in the electrolytic solution 13 in the form of bubbles without being dissolved.

In addition, although not particularly limited, an example of the reaction in the case where the reactant is methanol and the electrolyte is a brominated salt is shown in the following formula (C). In the case where the reactant is methanol and the electrolyte is a brominated salt, in the second reaction chamber 60Y, the oxidized product, bromine, reacts with methanol to produce dimethyl carbonate (DMC). Also, bromine is reduced to bromide ions to produce, for example, hydrogen bromide.

CO + Br₂ + CH₃OH → DMC + 2HBr (C)

As described above, in the present embodiment as well, the electrochemical reaction apparatus 60 can synthesize an oxygen-containing organic material using carbon dioxide as a raw material, despite its simple configuration. Also, in the electrochemical reaction apparatus 60, by circulating the electrolytic solution 63, the production rate of an oxygen-containing organic material can be enhanced.

Furthermore, in the present embodiment, the reduction reaction of carbon dioxide and the synthesis reaction of an oxygen-containing organic material are carried out in the reaction sections (first and second reaction sections), which are different from each other, thereby making it easier for the respective reactions to proceed, and an oxygen-containing organic material, such as a carbonyl compound, can be efficiently produced. Also, the oxidized product, such as halogen, produced in the first reaction chamber 60X (first reaction section) is delivered to the second reaction chamber 60Y (second reaction section) by the convection flow F. Accordingly, an increase in power consumption due to the oxidized product, such as halogen, in the first reaction chamber 60X (first reaction section) is prevented, and occurrence of a side reaction in the cathode 61 due to the oxidized product, such as halogen, can also be prevented. Therefore, the amount of the target product to be produced can be increased while keeping power consumption low.

Note that all of the carbon dioxide contained in the electrolytic solution 63 generally does not react after only single passing through the reaction chambers. Therefore, in the present embodiment as well, by circulating the electrolytic solution 63, the production rate of an oxygen-containing organic material, such as a carbonyl compound, can be enhanced.

Note that the first catalyst to be used in the sixth embodiment is as described in the first embodiment. As described in the first embodiment, there is no particular limitation on the second catalyst as long as it catalyzes the electrochemical reaction that synthesizes an oxygen-containing organic material, such as a carbonyl compound, from the carbon monoxide, and it may include a metal element. The details of the metal element are as described in the first embodiment. Also, specifically, the second catalyst may be an active particle-containing catalyst or may be a metal salt, but it is preferably an active particle-containing catalyst. The details of the active particle-containing catalyst and the metal salt are as described above.

Also, in the sixth embodiment, the metal element to be used in the second catalyst may be contained in the electrolytic solution in the form of metal ions. The metal ions may be formed by, for example, blending the above metal salt in the electrolytic solution.

In the second reaction chamber, the second catalyst may be dissolved in the electrolytic solution, but may not be dissolved, and the second catalyst that is not dissolved may be dispersed in the electrolytic solution. There is no particular limitation on the content of the second catalyst in the electrolytic solution, and it may be adjusted to the extent that the synthesis of an oxygen-containing organic material, such as a carbonyl compound, proceeds appropriately. The specific content of the second catalyst is, for example, 0.01 g or more and 100 g or less, preferably 0.05 g or more and 50 g or less, and more preferably 0.1 g or more and 20 g or less per 1 L of the electrolytic solution. Note that the content of the second catalyst is, in the case where the metal salt is a hydrate, the amount excluding the mass of water molecules in the hydrate.

### <Seventh embodiment>

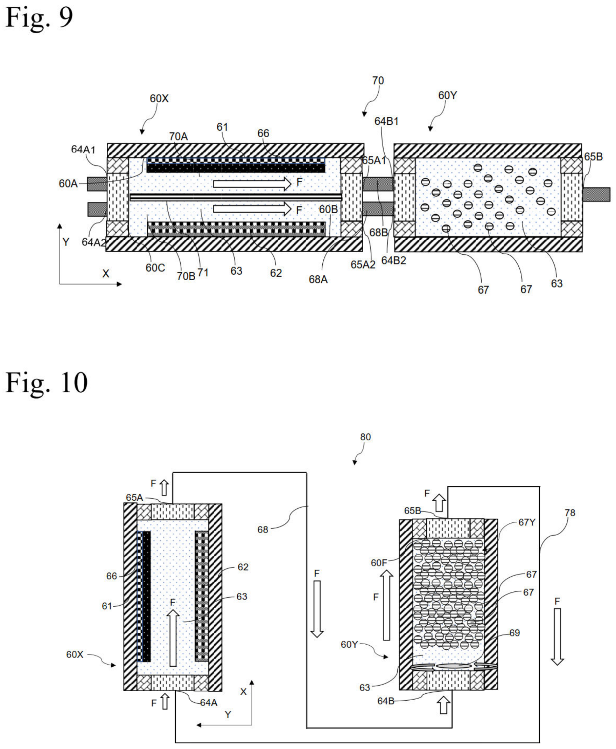

Fig. 9 shows an electrochemical reaction apparatus 70 according to the seventh embodiment. In the following, the points of difference in the seventh embodiment from the sixth embodiment will be described, and the parts where description is omitted are the same as in the sixth embodiment. The seventh embodiment differs from the sixth embodiment in that the first reaction chamber 60X of the electrochemical reaction apparatus 70 comprises an isolating layer 71. Note that, in the seventh embodiment, identical components to those in the sixth embodiment are marked with identical symbols.

The isolating layer 71 is arranged between the cathode 61 (that is, the first catalyst 66) and the anode 62, and is provided inside the first reactor 60X (electrochemical cell) so as to isolate the cathode 61 side region (cathode compartment 70A) from the anode 62 side region (anode compartment 70B). The configuration of the isolating layer 71 is the same as the isolating layer 41 in the fourth embodiment, and it may be one that isolates the carbon monoxide generated by the first catalyst 66 (cathode 61) from an oxidized product generated by the anode 62, and is preferably an ion exchange membrane.

Also, in the present embodiment, two first supply paths 64A1 and 64A2 and two first discharge ports 65A1 and 65A2 are provided in the first reactor 60X, while two second supply ports 64B1 and 64B2 are provided in the second reaction chamber 60Y as well. Then, each of the second supply ports 64B1 and 64B2 is connected to the first discharge ports 65A1 and 65A2 via connecting paths 68A and 68B, respectively.

With the above configuration, in the first reaction chamber 60X, the electrolytic solution 63 is allowed to flow from the first supply port 64A1 to the second discharge port 65A1 in the cathode 61 side region, which is isolated by the isolating layer 71. As a result, the electrolytic solution 63 is allowed to flow along the cathode 61 surface while in contact with the cathode 61 (first catalyst 66). On the other hand, the electrolytic solution 63 is allowed to flow from the first supply port 64A2 to the second discharge port 65A2 in the anode 62 side region, which is isolated by the isolating layer 71. As a result, the electrolytic solution 63 is allowed to flow along the anode 62 surface while in contact with the anode 62. Then, the electrolytic solution 63 flows in the order from each of the cathode 61 side region and the anode 62 side region to the second reaction chamber 60Y.

Accordingly, in the present embodiment as well, a reduced product of carbon dioxide (carbon monoxide) is produced at the cathode 61, while an oxidized product (for example, a redox mediator such as halogen), which is an active intermediate species, is produced at the anode 62. The carbon monoxide and active intermediate species produced in the first reaction chamber 60X are supplied to the second reaction section (second reaction chamber 60Y) by the convection flow F together with the electrolytic solution 63. Then, in the second reaction section (second reaction chamber 60Y), an oxygen-containing organic material, such as a carbonyl compound, is produced from the carbon monoxide, active intermediate species, and reactant by the action of the second catalyst 67.

In the present embodiment as well, the reduction reaction of carbon dioxide and the synthesis reaction of an oxygen-containing organic material are carried out in the reaction chambers (first and second reaction sections), which are different from each other, thereby enabling efficient production of an oxygen-containing organic material, such as a carbonyl compound. Also, the oxidized product, such as halogen, produced on the anode 62 side is delivered to the second reaction chamber 60Y (second reaction section) by the convection flow F, thereby preventing an increase in power consumption due to the oxidized product, such as halogen. Therefore, the amount of the target product to be produced can be increased while keeping power consumption low.

Furthermore, the isolating layer 71 does not allow the carbon monoxide produced in the cathode 61 side region to permeate to the anode 62 side, and does not allow the oxidized product (redox mediator or the like) generated on the anode 62 side to permeate to the cathode 21 side. Therefore, in the same manner as in the fourth embodiment, the electrochemical reaction apparatus 70 can synthesize an oxygen-containing organic material more efficiently using carbon dioxide as a raw material.

Accordingly, in the present embodiment as well, the amount of the target product to be produced can be increased while keeping power consumption low. Also, in the present embodiment as well, by circulating the electrolytic solution 13 in the same manner as in the first embodiment, the production rate of an oxygen-containing organic material can be further improved.

Also, in the present embodiment as well, by circulating the electrolytic solution 63, the production rate of an oxygen-containing organic material can be improved.

Note that the isolating layer 71 is provided in the above seventh embodiment, but a member other than the isolating layer 71 may be provided in place of the isolating layer 71. For example, a migration preventing layer may be provided in place of the isolating layer 71, that prevents migration of the carbon monoxide on the cathode 61 side to the anode 62 side and migration of the oxidized product on the anode 62 side to the cathode 61 side. As the migration preventing layer, the rectifying layer described in the third embodiment may be used, for example.

Also, in the present embodiment, the electrolytic solution 63 that contains carbon dioxide is supplied from both of the first supply ports 64A1 and 64A2, but the electrolytic solution 63 that is supplied from the supply port 64A2 to the anode 63 side region may be substantially free from carbon dioxide. By supplying the electrolytic solution 63 that is substantially free from carbon dioxide to the anode side region, it is so much easier to produce the oxidized product at the anode 62. Note that the electrolytic solution 63 that is substantially free from carbon dioxide means that the containment of carbon dioxide is permitted as long as its concentration is at the same level as that present in the atmosphere.

### <Eighth embodiment>

Fig. 10 shows an electrochemical reaction apparatus 80 according to the eighth embodiment. The eighth embodiment differs from the sixth embodiment in the configuration of the first reaction chamber and the second reaction chamber. In the following, the points of difference in the eighth embodiment from the sixth embodiment will be described, and the parts where description is omitted are the same as in the sixth embodiment. Note that, in the eighth embodiment, identical components to those in the sixth embodiment are marked with identical symbols.

In the eighth embodiment, the second reaction chamber 60Y is arranged so that the second supply port 64B is on the lower side and the second discharge port 65B is on the upper side, and as a result, a convection flow F in the second reaction chamber 60Y is formed so that the electrolytic solution 63 is allowed to flow from the below to the above along the vertical direction.

Similarly, the first reaction chamber 60X (electrochemical cell) is arranged so that the supply port 64A is on the lower side and the discharge port 65A is on the upper side, and as a result, a convection flow F is formed in the first reaction chamber 60X so that the electrolytic solution 63 is allowed to flow from the below to the above along the vertical direction.

Also, the second reaction chamber 60Y comprises a catalyst layer 67Y that comprises the second catalyst 67. The catalyst layer 67Y may be constituted by packing the second catalyst 67 inside the second reaction chamber 60Y in the form of a layer. The catalyst layer 67Y may be packed on a holding member such as a tray or a mesh inside the second reaction chamber 60Y, but no holding member may be provided.

As described above, the second catalyst 67 is preferably an active particle-containing catalyst in which active particles are supported on a support, but in the case where the catalyst layer 67Y is constituted as in the eighth embodiment, examples of the support include, as described above, porous carbon, silica, aluminum oxide, and zirconium oxide, among which silica, aluminum oxide, and zirconium oxide are preferable, and in particular, aluminum oxide is more preferable. In the case where the second catalyst 67 constitutes the catalyst layer 67Y as in the present embodiment, from the viewpoint of appropriately allowing the reaction to proceed in the second reaction chamber 60Y, the metal amount of the active particles is 0.1 parts by mass or more and 15 parts by mass or less, preferably 0.2 parts by mass or more and 10 parts by mass or less, and more preferably 0.3 parts by mass or more and 3 parts by mass or less, with respect to 100 parts by mass of the support.

Accordingly, the active particle-containing catalyst to be used in the catalyst layer 67Y as in the eighth embodiment may be produced as described above, but the support and the metal amount in the second catalyst raw material may be adjusted as appropriate so as to achieve the ratio described above.

The second reaction chamber 60Y may comprise, inside thereof, a gas diffusing mechanism 69 on the underside of the catalyst layer 67Y. The gas diffusing mechanism 69 may be provided above the second supply port 64B. Although there is no particular limitation on the gas diffusing mechanism 69 as long as it can diffuse the carbon monoxide contained in the electrolytic solution 63 that has been supplied from the second supply port 64B, for example, it may be a stirring device constituted by a stirring blade, a stirrer, or the like.

In the present embodiment, as described above, the electrolytic solution 63 that contains the carbon monoxide is distributed from the below to the above in the second reaction chamber 60Y, which enables the carbon monoxide in the form of bubbles in the electrolytic solution 63 to move smoothly due to buoyancy, and therefore, an oxygen-containing organic material, such as a carbonyl compound, can be efficiently synthesized in the catalytic layer 67Y. Also, provision of the gas diffusing mechanism 69 allows the carbon monoxide to be diffused even more appropriately in the electrolytic solution 63, and therefore, the production efficiency of an oxygen-containing organic material can be further increased.

Similarly, in the first reaction chamber 60X (electrochemical cell) as well, the electrolytic solution 63 is distributed from the below to the above, which efficiently reduces carbon dioxide to carbon monoxide in the catalyst 66.

Note that, in the present embodiment, the electrochemical reaction apparatus 80 is shown in which a circulating path 78 is provided that connects the second discharge port 65B of the second reaction chamber 60Y to the first supply port 64A of the first reaction chamber 60X, and the electrochemical solution 63 can be circulated, but in the present embodiment as well, the electrolytic solution 63 may not be circulated as in the above sixth and seventh embodiments, and others.

Also, in the eighth embodiment, the gas diffusing mechanism 69 may be omitted. Furthermore, in the second reaction chamber 60Y, the convection flow F is formed so that the electrolytic solution 63 is allowed to flow from the below to the above along the vertical direction, but there is no limitation to such a configuration, and the convection flow F may be formed so that electrolytic solution 63 is allowed to flow from the above to the below. Similarly, in the first reaction chamber 60X, the convection flow F need not be formed so that the electrolytic solution 63 is allowed to flow from the below to the above along the vertical direction, and the convection flow F may be formed so that the electrolytic solution 63 is allowed to flow from the above to the below.

### <Ninth embodiment>

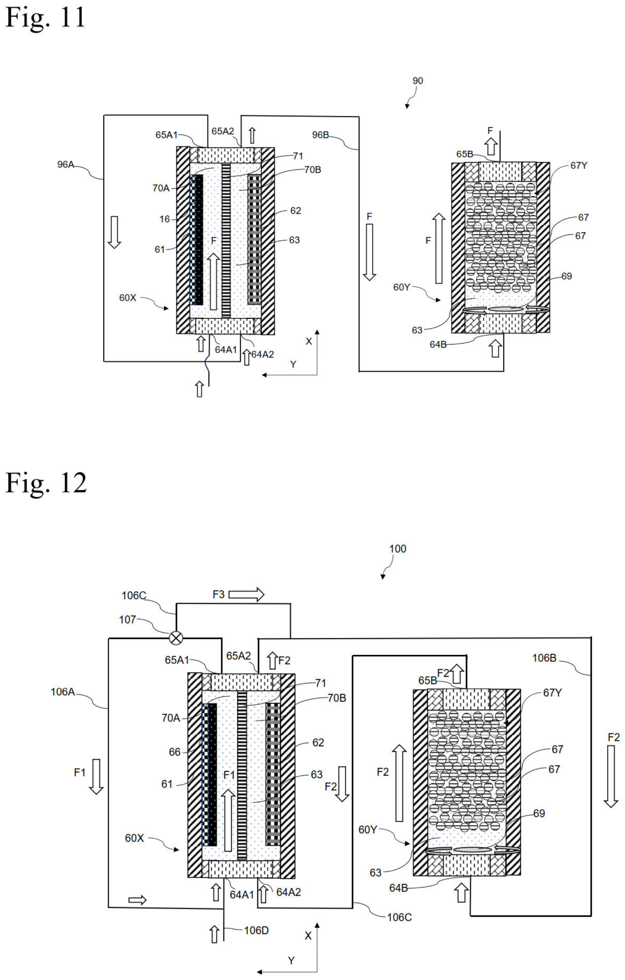

Fig. 11 shows an electrochemical reaction apparatus 90 according to the ninth embodiment. The ninth embodiment differs from the eighth embodiment in the configuration of the first reaction chamber and the flow direction of the convection flow F. In the following, the points of difference in the ninth embodiment from the eighth embodiment will be described in detail, and the parts where description is omitted are the same as in the first embodiment. Note that, in the ninth embodiment, identical components to those in the eighth embodiment are marked with identical symbols.

In the ninth embodiment, the configuration of the first reaction chamber 60X is the same as in the seventh embodiment, and accordingly, the first reaction chamber 60X is provided so that the cathode 61 side region (cathode compartment 70A) is isolated from the anode 62 side region (anode compartment 70B) by the isolating layer 71. Meanwhile, the second reaction chamber 60Y has the same configuration as the second reaction chamber 60Y in the eighth embodiment.

In the present embodiment, the first discharge port 65A1 of the cathode compartment 70A is connected to the first supply port 64A2 of the anode compartment 70B via a connecting path 96A. Also, the first discharge port 65A2 of the anode compartment is connected to the second supply port 64B of the second reaction chamber 60Y via a connecting path 96B. Therefore, the electrolytic solution 63 containing carbon dioxide that has been supplied from the supply port 64A1 of the cathode compartment 70A has a direction of the convection flow F so that it flows in the order of the cathode compartment 70A, the anode compartment 70B, and the second reaction chamber 60Y.

Accordingly, in the present embodiment as well, the amount of the target product to be produced can be increased while keeping power consumption low as in the seventh embodiment, and an oxygen-containing organic material can also be produced more efficiently by the provision of the partition wall layer 71.

Note that, in the present embodiment as well, the electrolytic solution 63 that has been discharged from the second discharge port 65B may be circulated and resupplied from the first supply port 64A via a circulating path not shown in the figure, and the circulation may be repeated.

### <Tenth embodiment>

Fig. 12 shows an electrochemical reaction apparatus 100 according to the tenth embodiment. The tenth embodiment differs from the ninth embodiment in that the form of convection flow of the electrolytic solution 63 is different. In the following, the points of difference in the tenth embodiment from the ninth embodiment will be described in detail, and the parts where description is omitted are the same as in the ninth embodiment. Note that, in the tenth embodiment, identical components to those in the ninth embodiment are marked with identical symbols.

In the present embodiment, the electrochemical reaction apparatus 100 comprises a circulating path 106A that connects the first discharge port 65A1 of the cathode compartment 70A to the first supply port 64A1. Therefore, the electrolytic solution 63 in the cathode compartment 70A is circulated by being discharged from the first discharge port 65A1 of the cathode compartment 70A and resupplied from the first supply port 64A1 through the circulating path 106A. In other words, the electrolytic solution 63 in the cathode compartment 70A is allowed to flow so as to form a convection flow F1 that circulates so that it exits from the first reaction chamber 60X and returns to the cathode compartment 70A again.

The electrochemical reaction apparatus 100 also comprises a circulating path 106B that connects the first discharge port 65A2 of the anode compartment 70B to the second supply port 64B of the second reaction chamber 60Y, and a circulating path 106C that connects the second discharge port 65B of the second reaction chamber 60Y to the first supply port 64A2 of the anode compartment 70B. Accordingly, the electrolytic solution 63 is allowed to flow between the anode compartment 70B and the second reaction chamber 60Y so as to form a convection flow F2 that circulates via the circulating paths 106B and 106C.

The electrochemical reaction apparatus 100 further comprises a branched path 106C that branches off from the circulating path 106A, and the first discharge port 65A1 of the cathode compartment 70A is also connected to the circulating path 106B via the circulating path 106A and the branched path 106C. As a result, a connection path is also formed that connects the first circulation path (circulating path 106A) through which the convection flow F1 flows and the second circulation path through which the convection flow F2 flows.

A valve 107 is provided at the connection section of the circulating path 106A and the branched path 106C. The valve 107 is a valve that, when opened, allows at least part of the gas in the electrolytic solution 63 (for example, gas that is not dissolved in the electrolytic solution 63) to flow out to the branched path 106C side. Accordingly, when the valve 107 is closed, all of the electrolytic solution 63 that has been discharged from the first discharge port 65A1 of the cathode compartment 70A is circulated through the first circulation path and returned to the cathode compartment 70A. Also, when the valve 107 is opened, part of the gas contained in the electrolytic solution 63 that passes through the circulating path 106A is supplied to the second circulation path (that is, the path circulating through the reaction section (first reaction chamber) 60Y and the anode compartment 70B) via the branched path 106C (that is, the connection path).

In other words, the electrolytic solution 63 that has been discharged from the discharge port 65A1 of the cathode compartment 70A can be switched between being allowed to flow by the convection flow F1 (also referred to as "circulation"), or being allowed to flow by the convection flow F1 while at least a part of the gas is allowed to flow by the convection flow F3 (also referred to as "opening"). The circulation and opening may be carried out repeatedly. Note that the gas that is allowed to flow by the convection flow F3 is mixed gas of carbon monoxide and carbon dioxide.

There is no particular limitation on the time for which the electrolytic solution 63 is allowed to flow by the circulation (also referred to as "circulation time"), but it may be, for example, about 1 to 20 minutes, preferably 2 to 15 minutes, and more preferably 3 to 10 minutes. By setting the circulation time at or above the lower limit value described above, a large amount of carbon dioxide can be reduced to carbon monoxide, while by setting the circulation time at or below the upper limit value described above, carbon dioxide can be reduced to carbon monoxide in a proportion that is commensurate with the circulation time. Therefore, by keeping the circulation time within the above-described range, the productivity of the target product can be improved.

Also, there is no particular limitation on the time for which the gas contained in the electrolytic solution 63 is allowed to flow with the convection flow F3 by the opening (also referred to as "opening time"), but from the viewpoint of improving productivity, for example, it may be shorter than the circulation time described above. The specific opening time may be, for example, about 0.5 to 15 minutes, preferably 1 to 10 minutes, and more preferably 2 to 8 minutes. By keeping the opening time within the above-described range, the productivity of the target product can be improved.

Also, a supply path 106D is further connected to the first supply port 64A1 of the first reaction chamber (electrochemical cell) 60X, and the electrolytic solution 63 that contains carbon dioxide may be supplied to the cathode compartment 70A via the supply path 106D and the first supply port 64A1. Furthermore, discharge paths, not shown in the figure, are connected to the second discharge port 65B of the second reaction chamber (second reaction section) 60Y, the first discharge port 65A2 of the anode compartment 70B, or other, and the oxygen-containing organic material produced in the electrochemical reaction apparatus 100 may be discharged from the second discharge port 65B, the first discharge port 65A2, or the other, together with the electrolytic solution 63, to the outside of the electrochemical reaction apparatus 100.

The electrochemical reaction apparatus 100 according to the present embodiment may be operated with the convection flows described above being switched. First, the electrochemical reaction apparatus 100 is maintained with the valve 107 closed, and the electrolytic solution 63 that contains carbon dioxide is circulated so that it repeatedly flows in and out of the cathode compartment 70A by the convection flow F1. Then, after a certain period of time of the circulation has elapsed, the valve 107 is opened, and as a result, the electrolytic solution 63 is circulated through the first circulation path and returned to the cathode compartment 70A, while a part of the gas contained therein (mixed gas of carbon monoxide and carbon dioxide) is supplied to the second circulation path (that is, the path that circulates through the second reaction chamber (second reaction section) 60Y and the anode compartment 70B) via the branched path 106C. In addition, in the second circulation path, the convection flow F2 is formed so that the electrolytic solution 63 circulates between the second reaction chamber 60Y and the anode compartment 70B. Accordingly, the second reaction chamber 60Y synthesizes an oxygen-containing organic material from the carbon monoxide that has been supplied via the branched path 106C and the oxidized product that has been generated at the anode. Then, the electrolytic solution 63 that contains the oxygen-containing organic material synthesized in the reaction chamber 60Y or the like may be discharged as appropriate to the outside via the discharge port 65B, the discharge port 65A2, or the other.

However, the electrolytic solution 63 that has been discharged via the discharge port 65B, the discharge port 65A2, or the other may be resupplied via the supply port 64A1. In other words, the electrolytic solution 63 may also circulate among the cathode compartment 70A, the anode compartment 70B, and the second reaction section 60Y.

Also, in the case where the electrolytic solution 63 is discharged from the discharge port 65B, the discharge port 65A2, or the other and the discharged electrolytic solution 63 is not resupplied from the supply port 64A1, the amount of the electrolytic solution contained in the electrochemical reaction apparatus 100 is reduced, and therefore, the electrolytic solution 63 that contains carbon dioxide may be additionally supplied via the supply port 64A1.

With the above operation performed, in the electrolytic solution 63 that is circulated in and out of the cathode compartment 70A repeatedly by the convection flow F1, the reduction reaction is repeatedly performed, and the carbon dioxide contained in the electrolytic solution 63 can be reduced to carbon monoxide at a high conversion rate. In addition, the gas contained in the electrolytic solution 63 is supplied to the second reaction chamber 60Y and the anode compartment 70B with an increased content rate of carbon monoxide. Therefore, in the present embodiment, an oxygen-containing organic material is produced as the electrolytic solution 63 circulates between the anode compartment 70B and the second reaction chamber 60Y in the second circulation path with an increased content rate of carbon monoxide in the contained gas, and therefore, its production efficiency can be increased.

### <Eleventh embodiment>

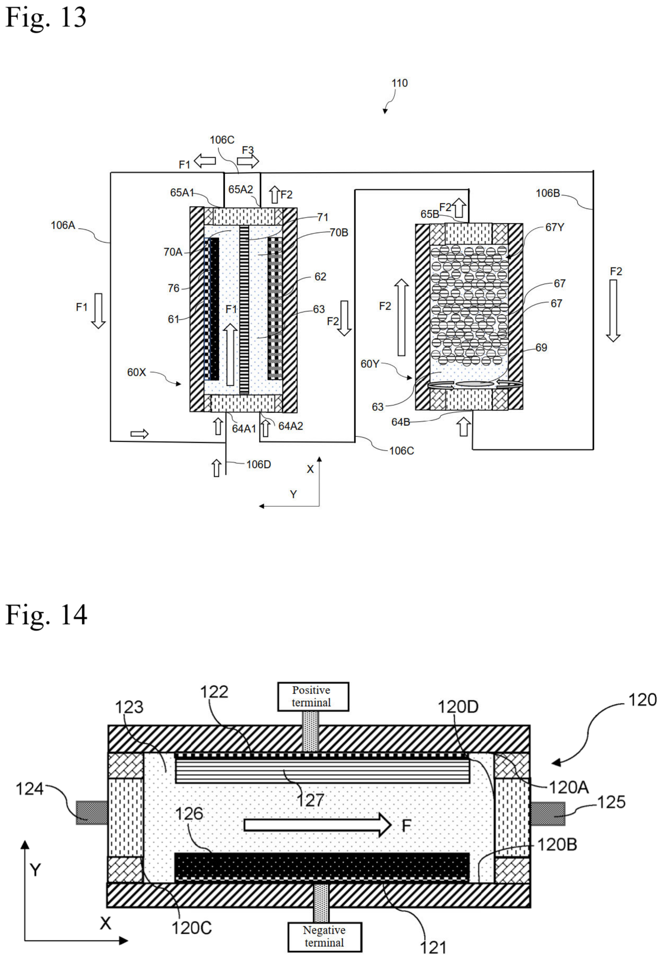

Fig. 13 shows an electrochemical reaction apparatus 110 according to the eleventh embodiment. In the following, the points of difference in the eleventh embodiment from the tenth embodiment will be described, and the parts where description is omitted are the same as in the tenth embodiment. In the tenth embodiment, the valve 107 is provided as described above, but in the present embodiment, the valve 107 is omitted. However, the connection section of the circulating path 106A and the branched path 106C has a valve structure that allows a part of the gas contained in the electrolytic solution 63 that passes through the circulating path 106A (for example, gas that is not dissolved in the electrolytic solution 63) to flow into the branched path 106C. Therefore, a part of the gas contained in the electrolytic solution 63 that passes through the circulating path 106A (gas that is not dissolved in the electrolytic solution 63) is always allowed to flow to the branched path 106C side at the connection section of the circulating path 106A and the branched path 106C.

Accordingly, the electrolytic solution 63 that has been discharged from the discharge port 65A1 of the cathode compartment 70A is circulated through the first circulation path (circulating path 106A) and returned to the cathode compartment 70A, while a part of the gas contained therein (mixed gas of carbon monoxide and carbon dioxide) is supplied to the second circulation path (that is, the second reaction chamber (second reaction section 60Y) and the anode compartment 70B) via the branched path 106C.

Therefore, in the present embodiment as well, in the electrolytic solution 63 that is circulated in and out of the cathode compartment 70A repeatedly by the convection flow F1, the reduction reaction is repeatedly performed, and the carbon dioxide contained in the electrolytic solution 63 can be reduced to carbon monoxide, as in the tenth embodiment. Also, the gas contained in the electrolytic solution 63 is mixed into the electrolytic solution that circulates through the second reaction chamber 60Y and the anode compartment 70B in a state where it contains carbon monoxide. Therefore, an oxygen-containing organic material is produced as the electrolytic solution 63 circulates between the anode compartment 70B and the second reaction chamber 60Y in the second circulation path, and therefore, the present embodiment can efficiently produce an oxygen-containing organic material as well.

### <Other embodiments>

In the above sixth to eleventh embodiments, aspects are shown in which the anode 62 does not contain the second catalyst, but the second catalyst may be contained in the anode as well, in addition to the second reaction section (second reaction chamber). When the second catalyst is also contained in the anode, an oxygen-containing organic material is produced on the anode as well as in the second reaction section, which can further enhance the production efficiency of the oxygen-containing organic material. Also, as for the oxidized product, such as halogen, produced at the anode, at least a part of it returns to a reduced species, such as halogen ions, when the oxygen-containing organic material is produced on the anode, which thus prevents an increase in power consumption and the occurrence of a side reaction due to the oxidized product, such as halogen.

Note that, in the case where the second catalyst is contained in the anode, it may be supported on the electrode substrate that constitutes the anode. Also, the second catalyst may be supported on the electrode substrate together with a catalyst additive and the like. Although there is no particular limitation on the method for supporting the second catalyst, or the second catalyst and catalyst additive (catalyst composition), on the electrode substrate, it is as described for the method for supporting the first catalyst on the electrode substrate, and the description therefor will thus be omitted.

In the sixth and seventh embodiments described above, aspects are shown in which the second catalyst is dispersed in the electrolytic solution in the second reaction section (second reaction chamber), but the second catalyst may constitute a catalyst layer in the same manner as in the eighth to eleventh embodiments. Alternatively, in the eighth to eleventh embodiments, the second catalyst may not constitute the catalyst layer, and may be dispersed in the electrolytic solution in the second reaction section (second reaction chamber) as shown in the sixth and seventh embodiments.

Also, in the first reaction chamber of the sixth to eleventh embodiments described above, a pair of the cathode and anode are arranged so that they face each other, but the arrangement of the cathode and anode is not limited to such an arrangement, and any arrangement is possible. For example, any of the arrangements described in the first to fifth embodiments may be employed. In addition, the number of cathodes and the number of anodes are not particularly limited in the sixth to eleventh embodiments either, and they do not have to be one, and may be two or more.

Note that, in each above embodiment, a redox species was used as the electrolyte in the electrolytic solution, but as for the electrolyte, an electrolyte other than the redox species may be contained in addition to the redox species.

Examples of the electrolyte other than the redox species include a perchlorate salt such as sodium perchlorate (NaClO₄), zinc perchlorate, barium perchlorate, lithium perchlorate, and ammonium perchlorate, sodium sulfate, potassium sulfate, and ammonium sulfate. One of these electrolytes other than the redox species may be used alone, or two or more thereof may be used in combination.

Also, for example, in the first to fifth embodiments, the redox species as the electrolyte may be omitted and the above-described electrolyte other than the redox species may be used in place of the redox species. In the first to fifth embodiments, an oxygen-containing organic material, such as a carbonyl compound, can be synthesized at a certain conversion rate even when the electrolyte other than the redox species is used in place of the redox species. Note that, in the case where the electrolyte other than the redox species is used in place of the redox species, the reaction of synthesizing an oxygen-containing organic material, such as a carbonyl compound, from the carbon monoxide and the reactant is not carried out in the presence of the oxidized species. Accordingly, the second catalyst synthesizes an oxygen-containing organic material, such as a carbonyl compound, from the carbon monoxide and the reactant without the use of the oxidized species.

Furthermore, the configuration of each of the embodiments described above is specifically described to illustrate the present invention, and the present invention is not limited to the configuration of each of the embodiments described above.

### Examples

Hereinafter, the present invention will be described in further detail with reference to Examples, but the present invention is not limited in any way by these Examples.

### (Example 1)

### [Preparation of catalysts]

38.0 mg of poly(4-vinylpyridine) (P4VP, weight-average molecular weight 60,000) was dispersed in 50 ml of ethanol to obtain a P4VP dispersion. 1.8 ml of a 20 mM Co(II)(NO₃)₂/ethanol solution (6.5 mg, Co = 2 mg) and 54 mg of ketjen black ("ECP600JD", manufactured by Lion Specialty Chemicals Co., Ltd.) were mixed in the P4VP dispersion and dried to obtain a powder (first catalyst raw material mixture). The cobalt content in the first catalyst raw material mixture was 2% by mass. The obtained powder was sintered in an argon atmosphere at 200°C for 3 hours to obtain a first catalyst (CoP4VP).

After dispersing 60 mg of Pd(NO₃)₂·2H₂O (manufactured by Sigma-Aldrich Co. LLC.) and 60 mg of ketjen black (product name "EC-300J" (Fuel Cell Store), BET specific surface area 800 m²/g, average primary particle diameter 40 nm) in 50 ml of ion exchanged water, the dispersion was dried and then heated at 300°C for 1 hour to obtain a second catalyst (Pd-C).

### [Preparation of electrodes]

By patterning a laminated body of FTO (current collecting layer: thickness about 2 µm)/glass with a size of 4 cm × 4 cm by laser processing, a base plate was obtained in which three rows of strip-shaped current collecting layers (1 cm × 4 cm) were formed. Two of these base plates were prepared.

24 mg of the first catalyst and 120 µL of a 10 mass% catalyst additive (Nafion) dispersion were mixed and dispersed in 4800 µL of acetone, sprayed on every other strip-shaped current collecting layer described above of each base plate, and dried to form catalyst layers including the first catalyst to obtain cathodes.

Also, 24 mg of the second catalyst and 120 µL of a 10 mass% catalyst additive (PTFE) dispersion were mixed and dispersed in 4800 µL of acetone, sprayed on the current collecting layers other than the current collecting layers where the cathodes had been formed, and dried to form catalyst layers including the second catalyst to obtain anodes.

### [Preparation of electrochemical reaction apparatus (cell)]

A 4 mm thick frame (made of ABS) provided with tube holes (supply port and discharge port) was prepared, and this was sandwiched between the base plates on which the cathodes and anodes had been formed, with the cathodes and anodes facing each other, via a Teflon (R) sheet (packing) of a thickness of 0.5 mm, which had been cut to the same size. By sealing it using an epoxy resin, the evaluation cell (electrochemical reaction apparatus) shown in Fig. 1 was created.

### [Evaluation of electrochemical reaction apparatus (cell)]

A 0.1 M LiBr solution in methanol was placed in a 50 ml solvent bottle and bubbled with carbon dioxide for 15 minutes to prepare an electrolytic solution in which the carbon dioxide was dissolved. Using a diaphragm pump to deliver and circulate the electrolytic solution at 5 ml/min, a current of 12 mA/cm² was applied between the cathodes and anodes for 15 minutes to cause an electrochemical reaction. The obtained electrolytic solution was analyzed by a gas chromatogram and the selectivity for the target product was calculated. The target product of each Example is described in Table 1.

### (Example 2)

### [Preparation of catalysts]

The first catalyst and the second catalyst were prepared in the same manner as in Example 1.

### [Preparation of electrodes]

Two base plates composed of a laminated body of FTO/glass (FTO coated surface 3 cm × 4 cm, thickness of FTO about 2 µm) having a size of 4 cm × 4 cm were prepared. 24 mg of the first catalyst and 120 µL of a 10 mass% catalyst additive (Nafion) dispersion were mixed and dispersed in 4800 µL of acetone, and sprayed on the FTO surface (current collector) of one base plate to form a catalyst layer including the first catalyst, thus preparing a cathode on the base plate. Similarly, 24 mg of ketjen black and 120 µL of a 10 mass% catalyst additive (Nafion) dispersion were mixed and dispersed in 4800 µL of acetone, and sprayed on the FTO surface (current collector) of the other base plate to form a catalyst layer including the second catalyst, thus preparing an anode on the base plate.

### [Preparation of electrochemical reaction apparatus]

A 4 mm thick frame (made of ABS) provided with tube holes (first supply port and first discharge port) was prepared, and this was sandwiched between the base plate having the anode and the base plate having the cathode, with the anode and cathode facing each other, via a Teflon (R) sheet (packing) of the same size, to prepare a first reaction section (first reaction chamber).

Next, glass plates of 2 cm × 2 cm and a 4 mm thick frame provided with tube holes (second supply port and second discharge port) were prepared, and this frame was sandwiched and held between the two glass plates via a Teflon (R) sheet (packing) that had been cut to the same size, thereby preparing a second reaction section (second reaction chamber). A membrane filter (pore size: 3 µm) was inserted into the tube hole at the back side (second discharge port) of the second reaction section. 10 mg of the second catalyst was dispersed in 1 ml of methanol, and the dispersion was fed into the second reaction section using a syringe through a syringe hole prepared otherwise. Also, by connecting the tube hole at the back side (first discharge port) of the first reaction section to the tube hole at the former side (second supply port) of the second reaction section, the electrochemical reaction apparatus shown in Fig. 8 was obtained.

### [Evaluation of electrochemical reaction apparatus (cell)]

A 0.1 M LiBr solution in methanol was placed in a 50 ml solvent bottle and bubbled with carbon dioxide for 15 minutes to adjust an electrolytic solution in which the carbon dioxide was dissolved. Using a diaphragm pump to deliver and circulate the electrolytic solution at 5 ml/min, a current of 12 mA/cm² was applied between the cathode and anode for 15 minutes to cause an electrochemical reaction. Furthermore, the second catalyst in the second reaction section was diffused using a stirrer. The obtained electrolytic solution was analyzed by a gas chromatogram and the selectivity for the target product was calculated.

### (Example 3)

The same process was performed as in Example 2, except that an ion exchange membrane (trade name. Nafion 117), which constitutes the isolating layer, was inserted in the first reaction section to isolate the anode side region from the cathode side region, as shown in Fig. 9.

### (Example 4)

### [Preparation of catalysts]

The first catalyst and the second catalyst were prepared in the same manner as in Example 1.

### [Preparation of electrodes]

Two sheets of carbon paper (trade name "Toray060", manufactured by Toray Industries, Inc.) cut to 1 cm × 4 cm were prepared. 8 mg of the first catalyst and 40 µL of a 10 mass% catalyst additive (Nafion) dispersion were mixed and dispersed in 1600 µL of acetone, and sprayed on one carbon paper to obtain a cathode on which a catalyst layer including the first catalyst had been formed. Also, 8 mg of the second catalyst and 40 µL of a 10 mass% catalyst additive (Nafion) dispersion were mixed and dispersed in 1600 µL of acetone, and sprayed on the other carbon paper to obtain an anode on which a catalyst layer including the second catalyst had been formed.

### [Preparation of electrochemical reaction apparatus (cell)]

Two housings made of vinyl chloride were prepared and the housings were processed to install a SUS current collecting rod, the anode or cathode, and a tube hole in each housing, as shown in Fig. 2. A 4 mm thick frame (polyvinyl chloride) was prepared, and this was sandwiched between the two housings via a Teflon (R) sheet (packing) with the same shape to prepare the evaluation cell (electrochemical reaction apparatus) shown in Fig. 2.

### [Evaluation of electrochemical reaction apparatus (cell)]

The evaluation was performed in the same manner as in Example 1.

### (Example 5)

The same process was performed as in Example 4, except that a rectifying layer was inserted between the anode and the cathode in the evaluation cell (electrochemical reaction apparatus), as shown in Fig. 3. As the rectifying layer, a 200 µm thick PTFE mesh (mesh size: 0.2 mm (short diameter) × 0.9 mm (long diameter), mesh shape: diamond shape) was used.

### (Example 6)

### [Preparation of catalysts and electrodes]

The preparation was performed in the same manner as in Example 4.

### [Preparation of electrochemical reaction apparatus (cell)]

Two housings made of vinyl chloride were prepared and the housings were processed to install a SUS current collecting rod, the anode or cathode, and tube holes in each housing, as shown in Fig. 5. A 4 mm thick frame (polyvinyl chloride) was prepared, and this was sandwiched between the two housings via a Teflon (R) sheet (packing) with the same shape to prepare the evaluation cell (electrochemical reaction apparatus) shown in Fig. 5. Note that, in the evaluation cell, an ion exchange membrane (trade name. Nafion 117), which constitutes the isolating layer, was inserted to isolate the anode side region from the cathode side region.

### [Evaluation of electrochemical reaction apparatus (cell)]

The evaluation was performed in the same manner as in Example 1.

### (Example 7)

### [Preparation of catalysts]

The first catalyst and the second catalyst were prepared in the same manner as in Example 1.

### [Preparation of electrodes]

Two sheets of carbon paper (trade name "Toray060", manufactured by Toray Industries, Inc.) cut to 1 cm × 4 cm were prepared. 8 mg of the first catalyst and 40 µL of a 10 mass% catalyst additive (Nafion) dispersion were mixed and dispersed in 1600 µL of acetone, and sprayed on the carbon paper to obtain a cathode on which a catalyst layer including the first catalyst had been formed.

Also, 12 mg of the second catalyst and 60 µL of a 10 mass% catalyst additive (Nafion) dispersion were mixed and dispersed in 1600 µL of acetone, and sprayed on the carbon paper to obtain a cathode on which a catalyst layer including the second catalyst had been formed.

### [Preparation of electrochemical reaction apparatus (cell)]

Two housings made of vinyl chloride were prepared and the housings were processed to install a SUS current collecting rod(s), the cathode and anode or the cathode, and tube holes in each housing, as shown in Fig. 6. A 4 mm thick frame (polyvinyl chloride) was prepared, and this was sandwiched between the two housings via a Teflon (R) sheet (packing) with the same shape to prepare the evaluation cell.

### (Example 8)

The same process was performed as in Example 3, except that the second catalyst used was prepared as follows.

### [Preparation of second catalyst (PdAu-C)]

After dispersing 25 mg of Pd(NO₃)₂·2H₂O (manufactured by Sigma-Aldrich Co. LLC.), 37 mg of HAuCl₄·3H₂O (manufactured by Sigma-Aldrich Co. LLC.), and 75 mg of ketjen black in 50 ml of ion exchanged water, the dispersion was dried and heated at 300°C for 2 hours to obtain a second catalyst (PdAu-C). In the second catalyst, the molar ratio of Pd and Au was 50:50.

### (Example 9)

The same process was performed as in Example 3, except that the first catalyst used was prepared as follows.

### [Preparation of first catalyst (Au/carbon)]

After dispersing 30 mg of HAuCl₄·3H₂O (manufactured by Sigma-Aldrich Co. LLC.) and 60 mg of ketjen black in 50 ml of ion exchanged water, the dispersion was dried and then heated at 300°C for 0.5 hours to obtain a first catalyst (Au/carbon) in which nano-gold particles were supported on carbon black.

### (Example 10)

The same process was performed as in Example 6, except that the second catalyst was changed to PdAu-C prepared in the same manner as in Example 8.

### (Example 11)

The same process was performed as in Example 6, except that the first catalyst was changed to Au/carbon prepared in the same manner as in Example 9.

### (Example 12)

The same process was performed as in Example 6, except that the electrolytic solution was changed from the 0.1 M LiBr solution in methanol to a 0.1 M ethylene glycol/acetonitrile (mixing ratio 50:50 (volume ratio)) mixed solution.

### (Example 13)

The same process was performed as in Example 6, except that the electrolytic solution was changed from the 0.1 M LiBr solution in methanol to a 0.1 M solution in ethanol.

### (Comparative Example 1)

### [Preparation of catalysts]

The first catalyst and the second catalyst were prepared in the same manner as in Example 1.

### [Preparation of electrodes]

Two sheets of carbon paper (trade name "Toray060", manufactured by Toray Industries, Inc.) cut to 1 cm × 4 cm were prepared. 8 mg of the first catalyst and 40 µL of a 10 mass% catalyst additive (Nafion) dispersion were mixed and dispersed in 1600 µL of acetone, and sprayed on one carbon paper to obtain a cathode on which a catalyst layer including the first catalyst had been formed. Also, 8 mg of the second catalyst and 40 µL of a 10 mass% catalyst additive (Nafion) dispersion were mixed and dispersed in 1600 µL of acetone, and sprayed on the other carbon paper to obtain an anode on which a catalyst layer including the second catalyst had been formed.

### [Preparation of cell]

Two housings made of vinyl chloride were prepared and the housings were processed to install a SUS current collecting rod and the anode or cathode in each housing, as shown in Fig. 14. A 4 mm thick frame (polyvinyl chloride) provided with tube holes (supply port and discharge port) was prepared, and this was sandwiched between the two housings via a Teflon (R) sheet (packing) with the same shape to prepare the evaluation cell shown in Fig. 14.

Note that, as shown in Fig. 14, the evaluation cell was provided with a cathode 121 having a first catalyst 126 and an anode 122 having a second catalyst 127 on wall surfaces 120B and 120A facing each other in the Y direction, respectively, and provided with a supply port 124 and a discharge port 125 on wall surfaces 120C and 120D facing each other in the X direction, respectively, and a convection flow Y was formed so as to cross a pair of the first catalyst 126 and the second catalyst 127 facing each other. As a result, the convection flow Y was formed so that an electrolytic solution 123 came into equal contact with the first catalyst 126 and the second catalyst 127.

**[Table 1]**

| | Reactant | Structure | | | Results | |
|---|---|---|---|---|---|---|
| | | Cathode | Second catalyst | Electrolyte | Product | Selectivity (%) |
| Example 1 | MeOH | CoP4VP/c arbon | Pd-C (supported on anode) | NaBr | DMC | 20 |
| Example 2 | MeOH | CoP4VP/c arbon | Pd-C (dispersed) | NaBr | DMC | 31 |
| Example 3 | MeOH | CoP4VP/c arbon | Pd-C (dispersed) | NaBr | DMC | 38 |
| Example 4 | MeOH | CoP4VP/c arbon | Pd-C (supported on anode) | NaBr | DMC | 25 |
| Example 5 | MeOH | CoP4VP/c arbon | Pd-C (supported on anode) | NaBr | DMC | 28 |
| Example 6 | MeOH | CoP4VP/c arbon | Pd-C (supported on anode) | NaBr | DMC | 32 |
| Example 7 | MeOH | CoP4VP/c arbon | Pd-C (supported on anode) | NaBr | DMC | 24 |
| Example 8 | MeOH | CoP4VP/c arbon | PdAu-C (dispersed) | NaBr | DMC | 40 |
| Example 9 | MeOH | Au/ carbon | Pd-C (dispersed) | NaBr | DMC | 28 |
| Example 10 | MeOH | CoP4VP/c arbon | PdAu-C (supported on anode) | NaBr | DMC | 35 |
| Example 11 | MeOH | Au/ carbon | Pd-C (supported on anode) | NaBr | DMC | 28 |
| Example 12 | Ethylene glycol | CoP4VP/c arbon | Pd-C (supported on anode) | NaBr | EC | 23 |
| Example 13 | EtOH | CoP4VP/c arbon | Pd-C (supported on anode) | NaBr | DEC | 25 |
| Comparative Example 1 | MeOH | CoP4VP/c arbon | Pd-C (supported on anode) | NaBr | DMC | 8 |

| | | | | | | |
|---|---|---|---|---|---|---|
| DMC = dimethyl carbonate, EC = ethylene carbonate DEC = diethyl carbonate | | | | | | |

As shown in Table 1, in Examples 1 to 13, the electrochemical cells (electrochemical reaction apparatuses) comprise a cathode having a first catalyst, an anode, an electrolytic solution containing a reactant and an electrolyte, and a second catalyst, where the electrolytic solution has a convection flow and the direction of the convection flow is formed so that it comes into contact with the first catalyst and the second catalyst in this order, and as a result, even with simple configurations, it was possible to produce carbonyl compounds at a selectivity at or above a certain level.

In contrast, in Comparative Example 1, the convection flow F of the electrolytic solution was formed so as to come into equal contact with the first catalyst and the second catalyst, and the convection flow Y was not formed so as to come into contact with the first catalyst and the second catalyst in this order, and as a result, it was not possible to produce a carbonyl compound at a selectivity at or above a certain level.

### (Example 14)

An electrochemical reaction apparatus was prepared in the same manner as in Example 2 and evaluated by the following method.

### [Evaluation of electrochemical reaction apparatus]

A 0.1 M NaBr solution in methanol was placed in a 50 ml solvent bottle and bubbled with carbon dioxide for 15 minutes to adjust an electrolytic solution in which the carbon dioxide was dissolved. Using a diaphragm pump to deliver and circulate the electrolytic solution at 15 ml/min, a current of 24 mA/cm² was applied between the cathode and anode for 15 minutes to cause an electrochemical reaction. Furthermore, the second catalyst in the reaction section was diffused using a stirrer. The obtained electrolytic solution was analyzed by a gas chromatogram and the selectivity for the target product (DMC) was calculated to be 31%.

### (Example 15)

The same process was performed as in Example 14, except that, in the first reaction section (electrochemical cell), an ion exchange membrane (trade name. Nafion 117), which constitutes the isolating layer, was inserted to isolate the anode side region from the cathode side region, and that the frame was provided with two first supply ports and two first discharge ports each, forming the electrochemical reaction apparatus shown in Fig. 9. The selectivity for the target product (DMC) was calculated to be 38%.

### (Comparative Example 2)

### [Preparation of catalysts]

The first catalyst and the second catalyst were prepared in the same manner as in Example 14. The same evaluation cell as in Comparative Example 1 was prepared and the convection flow F was formed so that the electrolytic solution 123 came into equal contact with the first catalyst 126 and the second catalyst 127. When applying a current in the same manner as in Example 14, the selectivity for the target product (DMC) was 8%.

### (Example 16)

### [Preparation of catalysts]

The first catalyst was prepared in the same manner as in Example 14. As for the second catalyst 0.14 g of PdCl₂ (manufactured by Sigma-Aldrich Co. LLC.) (metal amount: 0.085 g) and 20 g of alumina beads were dispersed in 50 ml of aqueous ammonia at a concentration of 26% by mass, and the dispersion was then dried and heated at 200°C for 3 hours to obtain the second catalyst (Pd-Al₂O₃).

### [Preparation of electrodes]

Using the first catalyst and second catalyst obtained as described above, the anode and the cathode were prepared on the base plates in the same manner as in Example 14.

### [Preparation of cell]

The electrochemical reaction apparatus was made to be the electrochemical reaction apparatus 80, which has the configuration shown in Fig. 10, except that the gas diffusing mechanism 69 was not provided and that the second reaction chamber 60Y was arranged so that the supply port 64B was on the upper side and the discharge port 65B was on the lower side, so that in the second reaction chamber 60Y, the electrolytic solution 63 flows from the above to the below along the vertical direction.

Note that, in the preparation of the electrochemical reaction apparatus 80, the second catalyst was packed into the second reaction chamber 60Y to form a catalyst layer.

### [Evaluation of cell]

A 0.1 M NaBr solution in methanol was placed in a 50 ml solvent bottle and bubbled with carbon dioxide for 15 minutes to adjust an electrolytic solution in which the carbon dioxide was dissolved. Using a diaphragm pump to deliver and circulate the electrolytic solution at 15 ml/min, a current of 12 mA/cm², 24 mA/cm², or 48 mA/cm² was each applied between the cathode and anode for 15 minutes to cause an electrochemical reaction. The selectivity for the target product (DMC) is shown in Table 2 below.

### (Example 17)

The first catalyst, second catalyst, and electrodes were prepared in the same manner as in Example 16. Also, the electrochemical reaction apparatus 90 shown in Fig. 11 was prepared in the same manner, except that the gas diffusing mechanism was not provided. Using an ion exchange membrane (trade name. Nafion 117) as the isolating layer, the electrochemical reaction apparatus 90 was prepared following Example 16, but the first and second reaction chambers were arranged so that the electrolytic solution 13 flows from bottom to top along the vertical direction.

**[Table 2]**

| | Cathode | Partition wall layer | Anode | Reaction section (catalyst layer) | | 12m A/cm2 | 24m A/cm2 | 48m A/cm2 |
|---|---|---|---|---|---|---|---|---|
| | Catalyst | | Catalyst | Support | Catalyst metal | DMC(%) | DMC(%) | DMC(%) |
| Example 16 | CoP4VP | Absent | PdAu | Al2O3 | Pd | 21.3 | 38.6 | 45.1 |
| Example 17 | CoP4VP | Present | PdAu | Al2O3 | Pd | 29.1 | 33.3 | 25 |

### (Examples 18 to 20)

The first catalyst, second catalyst, and electrodes were prepared in the same manner as in Example 17. Also, a cell was prepared so as to have the configuration of the electrochemical reaction apparatus shown in Fig. 12, except that no gas diffusing mechanism was provided. Specifically, the same process was performed as in Example 17, except that a valve was provided so as to be in the configuration shown in Fig. 12, and that tubes were connected so as to be in the configuration shown in Fig. 12.

### [Evaluation of cell]

A 0.1 M NaBr solution in methanol was placed in a 50 ml solvent bottle and bubbled with carbon dioxide for 15 minutes to adjust an electrolytic solution in which the carbon dioxide was dissolved. Using a diaphragm pump to deliver and circulate the electrolytic solution at 15 ml/min, a current of 12 mA/cm² was applied between the cathode and anode for 15 minutes to cause an electrochemical reaction. Note that, during the 15 minutes, with the circulation time and opening time shown in Table 3 below, the circulation and opening were repeated at the number of times shown in Table 3.

As described above, the circulation time is the time during which the electrolytic solution is allowed to flow by the convection flow F1, while the opening time is the time during which the gas in the electrolytic solution (mixed gas of carbon dioxide and carbon monoxide) is allowed to flow by F3. Note that the volume of the electrolytic solution allowed to flow by the convection flow F1 during the circulation was 15 ml/ min. The selectivity for the target product (DMC) in each Example is shown in Table 3 below.

**[Table 3]**

| | Circulation time (min) | Opening time (min) | Number of times of circulation and opening | DMC |
|---|---|---|---|---|
| Example 18 | 2 | 1 | 5 | 15% |
| Example 19 | 4.5 | 3 | 2 | 42% |
| Example 20 | 8 | 7 | 1 | 33% |

### (Example 21)

The first catalyst, second catalyst, and electrodes were prepared in the same manner as in Example 17. Also, a cell was prepared so as to have the configuration of the electrochemical reaction apparatus shown in Fig. 13, except that no gas diffusing mechanism was provided. Specifically, a cell was prepared in the same manner as in Example 4, except that tubes were connected so as to be in the configuration shown in Fig. 13.

The same process was performed as in Example 17, except that a current of 25 mA/cm² or 50 mA/cm² was applied to the obtained cell between the cathode and the anode for 15 minutes to cause an electrochemical reaction, and the Faraday efficiency (FE) for the target product (DMC) was evaluated. The results are shown in Table 4 below.

### (Example 22)

The same process was performed as in Example 21, except that, in the second reaction chamber 60Y, the electrolytic solution 63 was allowed to flow from the above to the below along the vertical direction. The results are shown in Table 4 below.

**[Table 4]**

| | 25mA/cm2 | 50mA/cm2 |
|---|---|---|
| | DMC(FE)(%) | DMC(FE)(%) |
| Example 21 | 85 | 63 |
| Example 22 | 32 | 33 |

The above electrochemical reaction apparatuses of Examples 14 to 22 comprise an electrochemical cell (first reaction section) that comprises a cathode having a first catalyst and an anode, a second reaction section having a second catalyst, and an electrolytic solution containing a reactant and an electrolyte, where the electrolytic solution has a direction of a convection flow that flows in the order of the electrochemical cell (first reaction section) and the second reaction section, and therefore, it was possible to increase the selectivity for the target product and the like. Also, as shown in Examples 16, 17, 21, and 22, it can be understood that even when the current density is increased, the selectivity of the target product and the like is at or above a certain level, and the amount of production can be increased while keeping power consumption low.

In contrast, as for the electrochemical reaction apparatus of Comparative Example 2, it was difficult to increase the selectivity for the target product since the electrolytic solution did not have a direction of a convection flow that flows in the order of the electrochemical cell and the reaction section.

### Reference Signs List

10, 20, 30, 30A, 40, 50 electrochemical reaction apparatus (electrochemical cell)
60, 70, 80, 90, 100, 110 electrochemical reaction apparatus
10X, 20X, 60X first reaction chamber (first reaction section, electrochemical cell)
10Y, 30Y, 60Y second reaction chamber (second reaction section)
11, 21, 51, 61 cathode
12, 22, 52, 62 anode
13, 23, 53, 63 electrolytic solution
14, 24, 34A, 34B, 54A, 54B, 64A, 64B, 64A1, 64A2, 64B1, 64B2 supply port
15, 25, 35A, 35B, 55A, 55B, 65A, 65B, 65A1, 65A2discharge port
16, 26, 56, 66 first catalyst
17, 27, 57, 67 second catalyst
31, 32 rectifying layer
41, 71 isolating layer
70A cathode compartment
70B anode compartment

## Claims

1. An electrochemical reaction apparatus comprising:
a cathode comprising a first catalyst that reduces carbon dioxide to carbon monoxide;
an anode;
an electrolytic solution comprising a reactant and an electrolyte; and
a second catalyst that synthesizes an oxygen-containing organic material from the carbon monoxide and the reactant,
the electrolytic solution having a convection flow, a direction of the convection flow formed so that the electrolytic solution comes into contact with the first catalyst and the second catalyst in this order.

2. The electrochemical reaction apparatus according to claim 1, wherein the first catalyst and the second catalyst are arranged on an identical plane, and the electrolytic solution has a convection flow that flows in parallel to the plane.

3. The electrochemical reaction apparatus according to claim 1 or 2, wherein the first catalyst and the second catalyst are arranged so that they face each other in the electrochemical reaction apparatus.

4. The electrochemical reaction apparatus according to claim 3, comprising a rectifying layer between the first catalyst and the second catalyst.

5. The electrochemical reaction apparatus according to claim 3, comprising an isolating layer between the first catalyst and the second catalyst that isolates the carbon monoxide generated by the first catalyst from an oxidized product generated by the anode.

6. The electrochemical reaction apparatus according to any one of claims 1 to 5, wherein the first catalyst and the second catalyst are arranged so that they are orthogonal or inclined.

7. The electrochemical reaction apparatus according to any one of claims 1 to 6, wherein the anode comprises the second catalyst.

8. The electrochemical reaction apparatus according to any one of claims 1 to 7, wherein the electrochemical reaction apparatus comprises a first reaction section that comprises the cathode and the anode and a second reaction section that comprises the second catalyst, and
the electrolytic solution has a direction of the convection flow that flows in an order of the first reaction section and the second reaction section.

9. The electrochemical reaction apparatus according to claim 8, comprising an isolating layer in the first reaction section that isolates the carbon monoxide generated by the first catalyst from an oxidized product generated by the anode.

10. The electrochemical reaction apparatus according to claim 8 or 9,
wherein an inside of the first reaction section is isolated into a cathode side region and an anode side region by the isolating layer, and
the electrolytic solution has a direction of the convection flow that flows in an order of the cathode side region, the anode side region, and the second reaction section.

11. The electrochemical reaction apparatus according to any one of claims 8 to 10,
wherein an inside of the first reaction section is isolated into a cathode side region and an anode side region by the isolating layer, and
the electrolytic solution has a direction of the convection flow that flows in an order from each of the cathode side region and the anode side region to the second reaction section.

12. The electrochemical reaction apparatus according to any one of claims 8 to 11,
wherein an inside of the first reaction section is isolated into a cathode side region and an anode side region by the isolating layer, and
the electrolytic solution has a convection flow that circulates so that the convection flow exits from the cathode side region to an outside of the first reaction section, and then returns to the cathode side region.

13. The electrochemical reaction apparatus according to any one of claims 8 to 12,
wherein an inside of the first reaction section is isolated into a cathode side region and an anode side region by the isolating layer, and
the electrolytic solution has a convection flow that circulates between the anode side region and the second reaction section.

14. The electrochemical reaction apparatus according to claim 13, wherein the electrolytic solution further has a convection flow allowing a gas in the electrolytic solution to flow from the cathode side region to a path through which the circulating convection flow flows, so that the gas is supplied to the second reaction section.

15. The electrochemical reaction apparatus according to any one of claims 8 to 14,
wherein the second reaction section comprises a catalyst layer that comprises the second catalyst, and
the electrolytic solution has a convection flow that flows from below to above in the second reaction section.

16. The electrochemical reaction apparatus according to claim 15, comprising a gas diffusing mechanism on an underside of the catalyst layer.

17. The electrochemical reaction apparatus according to any one of claims 8 to 16,
wherein the second reaction section comprises a catalyst layer that comprises the second catalyst, and
the second catalyst is an active particle-containing catalyst comprising a support and active particles that are supported on the support and contain a metal element, and
a metal amount of the active particles in the catalyst layer is 0.1 parts by mass or more and 15 parts by mass or less with respect to 100 parts by mass of the support.

18. The electrochemical reaction apparatus according to any one of claims 1 to 17, wherein the second catalyst is contained in the electrolytic solution.

19. The electrochemical reaction apparatus according to any one of claims 1 to 18, wherein the first reaction section is a one-compartment type electrochemical cell.

20. The electrochemical reaction apparatus according to any one of claims 1 to 19, wherein the first catalyst comprises CoO and a component derived from a pyridine derivative.

21. The electrochemical reaction apparatus according to any one of claims 1 to 20, wherein the second catalyst comprises at least one of Pd and Au.

22. The electrochemical reaction apparatus according to any one of claims 1 to 21, wherein the reactant is an alcohol-based compound.

23. The electrochemical reaction apparatus according to any one of claims 1 to 22, wherein the second catalyst is a catalyst that synthesizes a carbonyl compound from the carbon monoxide and the reactant.

24. A method of producing an oxygen-containing organic material in which an oxygen-containing organic material is synthesized from carbon dioxide in an electrochemical reaction apparatus comprising: a cathode comprising a first catalyst that reduces carbon dioxide to carbon monoxide; an anode; an electrolytic solution comprising a reactant and an electrolyte; and a second catalyst that synthesizes an oxygen-containing organic material from the carbon monoxide and the reactant,
a convection flow being formed in the electrolytic solution so that the electrolytic solution comes into contact with the first catalyst and the second catalyst in this order.

25. The method of producing an oxygen-containing organic material according to claim 24,
wherein the electrochemical reaction apparatus comprises a first reaction section that comprises the cathode and the anode and a second reaction section that comprises the second catalyst, and
a direction of the convection flow is formed in the electrolytic solution that flows in an order of the first reaction section and the second reaction section.
